# EUROPEAN PATENT SPECIFICATION

(11) **EP 3 064 587 B1**
(45) Date of publication and mention of the grant of the patent: **14.08.2019**
(21) Application number: 15158086.7
(22) Date of filing: 06.03.2015
(51) Int. Cl.: C12N 15/82, C12N 15/10

(54) **Library of synthetic promoters for coordinated gene expression in eukaryotic cells or organisms**
Bibliothek synthetischer Promotoren zur koordinierten Genexpression in eukaryotischen Zellen oder Organismen
Bibliothèque de promoteurs synthétiques permettant de coordonner l'expression génique dans des cellules eucaryotes ou des organismes

(43) Date of publication of application: 07.09.2016
(73) Proprietor: Leibniz-Institut für Pflanzenbiochemie (IPB), 06120 Halle (Saale) (DE)
(72) Inventor: Marillonnet, Sylvestre, 06126 Halle (Saale) (DE); Tissier, Alain, 06108 Halle (Saale) (DE)
(74) Representative: Blodig, Wolfgang

(56) References cited:
- WO-A2-2010/054348
- Tore Dehli ET AL: "Tunable Promoters in Synthetic and Systems Biology" In: "Reprogramming Microbial Metabolic Pathways", 1 January 2012 (2012-01-01), Springer Netherlands, Dordrecht, XP055206943, ISSN: 0306-0225 ISBN: 978-9-40-075055-5 vol. 64, pages 181-201, DOI: 10.1007/978-94-007-5055-5_9, * pages 185,187; figures 9.1,9.3b * * pages 191,194 *
- TORNOE J ET AL: "Generation of a synthetic mammalian promoter library by modification of sequences spacing transcription factor binding sites", GENE, ELSEVIER, AMSTERDAM, NL, vol. 297, no. 1-2, 4 September 2002 (2002-09-04), pages 21-32, XP027360658, ISSN: 0378-1119 [retrieved on 2002-09-04]
- RÖMER PATRICK ET AL: "A single plant resistance gene promoter engineered to recognize multiple TAL effectors from disparate pathogens", PROCEEDINGS OF THE NATIONAL ACADEMY OF SCIENCES, NATIONAL ACADEMY OF SCIENCES, US, vol. 106, no. 48, 1 December 2009 (2009-12-01), pages 20526-20531, XP002570746, ISSN: 0027-8424, DOI: 10.1073/PNAS.0908812106 [retrieved on 2009-11-12]
- KAY SABINE ET AL: "Detailed analysis of the DNA recognition motifs of the Xanthomonas type III effectors AvrBs3 and AvrBs3 Delta rep16", PLANT JOURNAL, BLACKWELL SCIENCE, OXFORD, GB, vol. 59, no. 6, 1 September 2009 (2009-09-01), pages 859-871, XP002555456, ISSN: 1365-313X, DOI: 10.1111/J.1365-313X.2009.03922.X [retrieved on 2009-06-22]
- BOCH JENS ET AL: "Breaking the Code of DNA Binding Specificity of TAL-Type III Effectors", SCIENCE, AMERICAN ASSOCIATION FOR THE ADVANCEMENT OF SCIENCE, US, vol. 326, no. 5959, 1 December 2009 (2009-12-01), pages 1509-1512, XP002570745, ISSN: 0036-8075, DOI: 10.1126/SCIENCE.1178811 -& J. BOCH ET AL: "Breaking the Code of DNA Binding Specificity of TAL-Type III Effectors", SCIENCE, vol. 326, no. 5959, 29 October 2009 (2009-10-29), pages 1509-1512, XP055082380, ISSN: 0036-8075, DOI: 10.1126/science.1178811
- RENÉ GEI[BETA]LER ET AL: "Transcriptional Activators of Human Genes with Programmable DNA-Specificity", PLOS ONE, vol. 6, no. 5, 1 January 2011 (2011-01-01) , page e19509, XP055153699, ISSN: 1932-6203, DOI: 10.1371/journal.pone.0019509
- BENJAMIN A. BLOUNT ET AL: "Rational Diversification of a Promoter Providing Fine-Tuned Expression and Orthogonal Regulation for Synthetic Biology", PLOS ONE, vol. 7, no. 3, 19 March 2012 (2012-03-19), page e33279, XP055206940, DOI: 10.1371/journal.pone.0033279
- FAHIM FARZADFARD ET AL: "Tunable and Multifunctional Eukaryotic Transcription Factors Based on CRISPR/Cas", ACS SYNTHETIC BIOLOGY, vol. 2, no. 10, 18 October 2013 (2013-10-18), pages 604-613, XP055194786, ISSN: 2161-5063, DOI: 10.1021/sb400081r
- ORLANDO DE LANGE ET AL: "From dead leaf, to new life: TAL effectors as tools for synthetic biology", THE PLANT JOURNAL, vol. 78, no. 5, 6 March 2014 (2014-03-06), pages 753-771, XP055206842, ISSN: 0960-7412, DOI: 10.1111/tpj.12431
- JOHN BLAZECK ET AL: "Promoter engineering: Recent advances in controlling transcription at the most fundamental level", BIOTECHNOLOGY JOURNAL, vol. 8, no. 1, 14 January 2013 (2013-01-14), pages 46-58, XP055206622, ISSN: 1860-6768, DOI: 10.1002/biot.201200120
- PATRICK RÖMER ET AL: "Promoter elements of rice susceptibility genes are bound and activated by specific TAL effectors from the bacterial blight pathogen, Xanthomonas oryzae pv. oryzae", NEW PHYTOLOGIST, vol. 187, no. 4, 19 March 2010 (2010-03-19), pages 1048-1057, XP055033479, ISSN: 0028-646X, DOI: 10.1111/j.1469-8137.2010.03217.x
- AARON W. HUMMEL ET AL: "Addition of transcription activator-like effector binding sites to a pathogen strain-specific rice bacterial blight resistance gene makes it effective against additional strains and against bacterial leaf streak", NEW PHYTOLOGIST, vol. 195, no. 4, 2 September 2012 (2012-09-02), pages 883-893, XP055206648, ISSN: 0028-646X, DOI: 10.1111/j.1469-8137.2012.04216.x
- ANNEKATRIN RICHTER ET AL: "Designer TALEs team up for highly efficient gene induction", NATURE METHODS, vol. 10, no. 3, 27 February 2013 (2013-02-27), pages 207-208, XP055206833, ISSN: 1548-7091, DOI: 10.1038/nmeth.2373
- FLORIAN LIENERT ET AL: "Synthetic biology in mammalian cells: next generation research tools and therapeutics", NATURE REVIEWS MOLECULAR CELL BIOLOGY, vol. 15, no. 2, 17 January 2014 (2014-01-17), pages 95-107, XP055206837, ISSN: 1471-0072, DOI: 10.1038/nrm3738
- JENS BOCH ET AL: "TAL effectors - pathogen strategies and plant resistance engineering", NEW PHYTOLOGIST, vol. 204, no. 4, 26 August 2014 (2014-08-26), pages 823-832, XP055206619, ISSN: 0028-646X, DOI: 10.1111/nph.13015
- Tom Ellis ET AL: "Diversity-based, model-guided construction of synthetic gene networks with predicted functions", Nature Biotechnology, vol. 27, no. 5, 1 May 2009 (2009-05-01), pages 465-471, XP055107449, ISSN: 1087-0156, DOI: 10.1038/nbt.1536
- Tom Ellis ET AL: "Supplementary Information: Diversity-based, model-guided construction of synthetic gene networks with predicted functions", Nature Biotechnology (advance online publication), vol. 27, no. 5, 19 April 2009 (2009-04-19) , pages 1-30, XP055459235, ISSN: 1087-0156, DOI: 10.1038/nbt.1536

### SEQUENCE LISTING

<110> Leibniz-Institut für Pflanzenbiochemie
<120> Library of synthetic promoters for coordinated gene expression in eukaryotic cells or organisms
<130> EAD-16714
<160> 52
<170> PatentIn version 3.5
<210> 1
   <211> 97
   <212> DNA
   <213> Artificial Sequence
<220>
   <223> promoter sequence
<400> 1
   ggaggttatc acacttggta atttccccgc atagctgaac atctatacaa cactcatcgc    60
   gcggagaaac gcgcaacaaa ttggaggcgc attaatg                              97
<210> 2
   <211> 97
   <212> DNA
   <213> Artificial Sequence
<220>
   <223> promoter sequence
<400> 2
   ggagcttacg tagcagcacg gcctccccgc atagctgaac atctatataa atcttagccc 60
   gactgcaccg tgaggaaaag agggtttata tataatg 97
<210> 3
   <211> 98
   <212> DNA
   <213> Artificial Sequence
<220>
   <223> promoter sequence
<400> 3
   ggaggtcgtc aaatgataag tactccccgc atagctgaac atctatataa cgcagcaaat 60
   ggttgaaacg tattagcaac agctaaacct tcgaatgt 98
<210> 4
   <211> 97
   <212> DNA
   <213> Artificial Sequence
<220>
   <223> promoter sequence
<400> 4
   ggaggcgtgt cgttttagtg aggtccccgc atagctgaac atctatataa ggttttgcta 60
   ttcattgaaa gcagtagtga ctgatttgta tataatg 97
<210> 5
   <211> 96
   <212> DNA
   <213> Artificial Sequence
<220>
   <223> promoter sequence
<400> 5
   ggaggttctg tgggtttttt gggtccccgc atagctgaac atctatataa tgagaaacag 60
   gggcaaatcc ccgaaaacaa ataacgcgaa gcaatg 96
<210> 6
   <211> 97
   <212> DNA
   <213> Artificial Sequence
<220>
   <223> promoter sequence
<400> 6
   ggagatgtgc catggtcggg ggctccccgc atagctgaac atctatataa tcggaatgaa 60
   ttgcaaatag ggatgttata gggttatctc ggcaatg 97
<210> 7
   <211> 97
   <212> DNA
   <213> Artificial Sequence
<220>
   <223> promoter sequence
<400> 7
   ggagagatga ttgttcgtaa tattccccgc atagctgaac atctatataa gagacgagct 60
   accacgataa gcgcagcaaa agacacatac gttaatg 97
<210> 8
   <211> 97
   <212> DNA
   <213> Artificial Sequence
<220>
   <223> promoter sequence
<400> 8
   ggaggtgcct ttttaaaagg gtttccccgc atagctgaac atctatataa gaaacacgga 60
   cgccgaaacc cccccgccta gttgactctc cggaatg 97
<210> 9
   <211> 96
   <212> DNA
   <213> Artificial Sequence
<220>
   <223> promoter sequence
<400> 9
   gagtgtgaac gtggtgatgt ggtccccgca tagctgaaca tctatataaa aactgtaaca 60
   gaactgctgc tcaactagtc tccaactata taaatg 96
<210> 10
   <211> 97
   <212> DNA
   <213> Artificial Sequence
<220>
   <223> promoter sequence
<400> 10
   ggaggtaagt tctggcctct gggtccccgc atagctgaac atctatataa taggacagta 60
   agatatgcca gacaatgtag cacgtagaac ggaaatg 97
<210> 11
   <211> 96
   <212> DNA
   <213> Artificial Sequence
<220>
   <223> promoter sequence
<400> 11
   ggagcacctt ggaaaacagg tggtccccgc atagctgaac atctatataa gaaattagta 60
   actacgcttt caaagacatc ttaattaatt agaatg 96
<210> 12
   <211> 97
   <212> DNA
   <213> Artificial Sequence
<220>
   <223> promoter sequence
<400> 12
   ggagagtggg cgcgtttggt aagtccccgc atagctgaac atctatataa ataagctgcc 60
   tacgatttag cgcgacacaa ttcgacggtt ggaaatg 97
<210> 13
   <211> 97
   <212> **DNA**
   <213> Artificial Sequence
<220>
   <223> promoter sequence
<400> 13
   ggaggccgtc gggggggggt ctgtccccgc atagctgaac atctatataa taccggttat 60
   ccagcgacaa ccgtcaataa cttaacattg aacaatg 97
<210> 14
   <211> 97
   <212> DNA
   <213> Artificial Sequence
<220>
   <223> promoter sequence
<400> 14
   ggagttaagt aggctgttct tattccccgc atagctgaac atctatataa atagctcagt 60
   tgccttaaac gtagacagtc tattacacta ataaatg 97
<210> 15
   <211> 97
   <212> DNA
   <213> Artificial Sequence
<220>
   <223> promoter sequence
<400> 15
   ggaggtttat actttctcta atttccccgc atagctgaac atctatataa actggaactt 60
   tcactgacct gacagtgctt tcattgacgc gcgaatg 97
<210> 16
   <211> 97
   <212> DNA
   <213> Artificial Sequence
<220>
   <223> promoter sequence
<400> 16
   ggagaagctc acctgcatcc ttgtccccgc atagctgaac atctatataa aatttataca 60
   tttaaaccaa accctataaa atttctgtcc gcgaatg 97
<210> 17
   <211> 97
   <212> DNA
   <213> Artificial Sequence
<220>
   <223> promoter sequence
<400> 17
   ggagtaatgt acagcttcgc ggctccccgc atagctgaac atctatataa taactgttct 60
   acatactctc ggataaataa cgcaacttaa tataatg 97
<210> 18
   <211> 97
   <212> DNA
   <213> Artificial Sequence
<220>
   <223> promoter sequence
<400> 18
   ggagtcccgg atcctggttt cactccccgc atagctgaac atctatataa aacaaatctc 60
   gtcaagcaaa ccaattacga tggtccggga aggaatg 97
<210> 19
   <211> 97
   <212> DNA
   <213> Artificial Sequence
<220>
   <223> promoter sequence
<400> 19
   ggaggggtgg gggggttacc gtctccccgc atagctgaac atctatataa aaccaatcga 60
   cgactcattg gaatgcgtcc tgctccaaat agcaatg 97
<210> 20
   <211> 97
   <212> DNA
   <213> Artificial Sequence
<220>
   <223> promoter sequence
<400> 20
   ggagctgcaa tgcttactgt tattccccgc atagctgaac atctatataa aggttgagaa 60
   gtggcacttg caataattgc cgggcccatg ggaaatg 97
<210> 21
   <211> 97
   <212> DNA
   <213> Artificial Sequence
<220>
   <223> promoter sequence
<400> 21
   ggagagaggt gggtcgcggg cattccccgc atagctgaac atctatataa aaagcagttc 60
   atacgggaac gcagactagt cacgaaattc cgcaatg 97
<210> 22
   <211> 97
   <212> DNA
   <213> Artificial Sequence
<220>
   <223> promoter sequence
<400> 22
   ggaggcgcgt ttgattgggg gggtccccgc atagctgaac atctatataa aactgataac 60
   ttagtcagac tagcaaatgc tcacaataca aagaatg 97
<210> 23
   <211> 97
   <212> DNA
   <213> Artificial Sequence
<220>
   <223> promoter sequence
<400> 23
   ggaggaaccg cggttcggga gcgtccccgc atagctgaac atctatataa caggggcggg 60
   atatgatata gaaacaccag ggttagtaac aagaatg 97
<210> 24
   <211> 97
   <212> DNA
   <213> Artificial Sequence
<220>
   <223> promoter sequence
<400> 24
   ggagcaatgg cagaggggta tggtccccgc atagctgaac atctatataa aattgggacc 60
   tatgataatg ctgtagaacc tcataaatga tggaatg 97
<210> 25
   <211> 97
   <212> DNA
   <213> Artificial Sequence
<220>
   <223> promoter sequence
<400> 25
   ggagggtttg ttaccgtgta ttctccccgc atagctgaac atctatataa cgagcggtcg 60
   aaagcttcaa aattcaatga gtaagacccc aacaatg 97
<210> 26
   <211> 97
   <212> DNA
   <213> Artificial Sequence
<220>
   <223> promoter sequence
<400> 26
   ggagtcttgc ccatactcag ttctccccgc atagctgaac atctatataa tgtctctcga 60
   atgacaccga tacttcgatt tagcaggctg cccaatg 97
<210> 27
   <211> 97
   <212> DNA
   <213> Artificial Sequence
<220>
   <223> promoter sequence
<400> 27
   ggaggcatta cgggcgtagt ttgtccccgc atagctgaac atctatataa tcgaccagag 60
   caacgttctc gcctaggcgc gccttaccgg gaaaatg 97
<210> 28
   <211> 97
   <212> DNA
   <213> Artificial Sequence
<220>
   <223> promoter sequence
<400> 28
   ggaggtatcc cgagggcgaa tggtccccgc atagctgaac atctatataa ggcatctggt 60
   tagcaagcct caccagccgt gaacaatata atcaatg 97
<210> 29
   <211> 97
   <212> DNA
   <213> Artificial Sequence
<220>
   <223> promoter sequence
<400> 29
   ggagtcagag tgtggtcgtg ttttccccgc atagctgaac atctatataa gtttacacct 60
   gcccgaccta ttccttgatg agctcgcgca ccgaatg 97
<210> 30
   <211> 97
   <212> DNA
   <213> Artificial Sequence
<220>
   <223> promoter sequence
<400> 30
   ggagcactta ggttaactgc gcgtccccgc atagctgaac atctatataa ggaacagacg 60
   gctaacccat caatggaaca acgaacggcc cataatg 97
<210> 31
   <211> 97
   <212> DNA
   <213> Artificial Sequence
<220>
   <223> promoter sequence
<400> 31
   ggagtggttt tagcacagac aactccccgc atagctgaac atctatataa ggaaccgact 60
   ctctcccgcc cccgacacag gaccccctct ggtaatg 97
<210> 32
   <211> 97
   <212> DNA
   <213> Artificial Sequence
<220>
   <223> promoter sequence
<400> 32
   ggagcatgct ggggttggag gggtccccgc atagctgaac atctatataa cccggcccct 60
   caattaaccc tgctcaaagc caaagcaaca atcaatg 97
<210> 33
   <211> 97
   <212> DNA
   <213> Artificial Sequence
<220>
   <223> promoter sequence
<400> 33
   ggagtgggct gagggggacg ggttccccgc atagctgaac atctatataa cccaacgtga        60
   gatcacgttt acaatactcg tcttgactga cacaatg                                 97
<210> 34
   <211> 98
   <212> DNA
   <213> Artificial Sequence
<220>
   <223> promoter sequence
<400> 34
   ggagggcatg aggttagcta gtttccccgc atagctgaac atctatataa tgaatgtgtg        60
   agaagctttg acaagcttcg cccagcggcc ctaaatgt                                98
<210> 35
   <211> 97
   <212> DNA
   <213> Artificial Sequence
<220>
   <223> promoter sequence
<400> 35
   ggagatattc ctcggctgtc acttccccgc atagctgaac atctatataa tcaaattcct        60
   aggggctgcc atggttaccc cttgctatgt agcaatg                                 97
<210> 36
   <211> 96
   <212> DNA
   <213> Artificial Sequence
<220>
   <223> promoter sequence
<400> 36
   gagtgcgggg agttggtatg ggtccctgca tagctgaaca tctatataaa taacggccct        60
   gagtgtcgct agcaaatgca ccaaaccaac tcaatg                                  96
<210> 37
   <211> 97
   <212> DNA
   <213> Artificial Sequence
<220>
   <223> promoter sequence
<400> 37
   ggagcgatat gtttaatcgg cattccccgc atagctgaac atctatataa atccgcgcct        60
   taaatacggt tgtttgccta ttttgagcga gtaaatg                                   97
<210> 38
   <211> 97
   <212> DNA
   <213> Artificial Sequence
<220>
   <223> promoter sequence
<400> 38
   ggagtattgg tccacttgct ccgtccccgc atagctgaac atctatataa cgcagaaacc        60
   tcgcaactga cgttccgatt gcgataaaca cacaatg                                   97
<210> 39
   <211> 97
   <212> DNA
   <213> Artificial Sequence
<220>
   <223> promoter sequence
<400> 39
   ggagttgtaa actccaggtt agatccccgc atagctgaac atctatataa atcgcccatt        60
   gcccaggaca cgcaaccctg caacaatcag tacaatg                                   97
<210> 40
   <211> 97
   <212> DNA
   <213> Artificial Sequence
<220>
   <223> promoter sequence
<400> 40
   ggagcccttg ctatgacgat gcctccccgc atagctgaac atctatataa aaattccata        60
   acgtacagaa cgaagaacgg acccatatac aataatg                                   97
<210> 41
   <211> 97
   <212> DNA
   <213> Artificial Sequence
<220>
   <223> promoter sequence
<400> 41
   ggaggcgcgg gcaggggttg ggttccccgc atagctgaac atctatataa tcacccaacg 60
   ccgcatgcaa caactagtaa caaggccatt gagaatg 97
<210> 42
   <211> 97
   <212> DNA
   <213> Artificial Sequence
<220>
   <223> promoter sequence
<400> 42
   ggagcttttt acggggcacc tgttccccgc atagctgaac atctatataa caaaataaca 60
   gattgtctgt ccaagaggca gacattcgtt cataatg 97
<210> 43
   <211> 19
   <212> DNA
   <213> Artificial Sequence
<220>
   <223> primer
<400> 43
   cgatccaaag atgggacga 19
<210> 44
   <211> 26
   <212> DNA
   <213> Artificial Sequence
<220>
   <223> primer
<400> 44
   catctctttt ttcgcatagt agatgt 26
<210> 45
   <211> 27
   <212> DNA
   <213> Artificial Sequence
<220>
   <223> primer
<400> 45
   cctccacaga gacattctta tagtttc 27
<210> 46
   <211> 24
   <212> DNA
   <213> Artificial Sequence
<220>
   <223> primer
<400> 46
   tcaaagtcaa acttaggcaa gatg 24
<210> 47
   <211> 18
   <212> DNA
   <213> Artificial Sequence
<220>
   <223> primer
<400> 47
   agcatctccg ctggtctt 18
<210> 48
   <211> 20
   <212> DNA
   <213> Artificial Sequence
<220>
   <223> primer
<400> 48
   cgagtcaagg aatcctgcat 20
<210> 49
   <211> 57
   <212> DNA
   <213> Artificial Sequence
<220>
   <223> Talpro7
<220>
   <221> misc_feature
   <222> (19)..(37)
   <223> n is a, c, g, or t
<400> 49
   tttggtctca acatggagnn nnnnnnnnnn nnnnnnntcc ccgcatagct gaacatc 57
<210> 50
   <211> 87
   <212> DNA
   <213> Artificial Sequence
<220>
   <223> Talpro8
<220>
   <221> misc_feature
   <222> (19)..(61)
   <223> n is a, c, g, or t
<400> 50
   tttggtctca acaacattnn nnnnnnnnnn nnnnnnnnnn nnnnnnnnnn nnnnnnnnnn 60
   nttatataga tgttcagcta tgcgggg 87
<210> 51
   <211> 34
   <212> PRT
   <213> Artificial Sequence
<220>
   <223> TAL effector consensus
<220>
   <221> misc_feature
   <222> (12)..(13)
   <223> Xaa can be any naturally occurring amino acid
<400> 51
   Leu Thr Pro Glu Gln Val Val Ala Ile Ala Ser Xaa Xaa Gly Gly Lys
   1 5 10 15
   Gln Ala Leu Glu Thr Val Gln Arg Leu Leu Pro Val Leu Cys Gln Ala
   20 25 30
   His Gly
<210> 52
   <211> 5087
   <212> DNA
   <213> Artificial Sequence
<220>
   <223> pICH74043
<400> 52
   tcgacaaaat ttagaacgaa cttaattatg atctcaaata cattgataca tatctcatct 60
   agatctaggt tatcattatg taagaaagtt ttgacgaata tggcacgaca aaatggctag 120
   actcgatgta attggtatct caactcaaca ttatacttat accaaacatt agttagacaa 180
   aatttaaaca actatttttt atgtatgcaa gagtcagcat atgtataatt gattcagaat 240
   cgttttgacg agttcggatg tagtagtagc cattatttaa tgtacatact aatcgtgaat 300
   agtgaatatg atgaaacatt gtatcttatt gtataaatat ccataaacac atcatgaaag 360
   acactttctt tcacggtctg aattaattat gatacaattc taatagaaaa cgaattaaat 420
   tacgttgaat tgtatgaaat ctaattgaac aagccaacca cgacgacgac taacgttgcc 480
   tggattgact cggtttaagt taaccactaa aaaaacggag ctgtcatgta acacgcggat 540
   cgagcaggtc acagtcatga agccatcaaa gcaaaagaac taatccaagg gctgagatga 600
   ttaattagtt taaaaattag ttaacacgag ggaaaaggct gtctgacagc caggtcacgt 660
   tatctttacc tgtggtcgaa atgattcgtg tctgtcgatt ttaattattt ttttgaaagg 720
   ccgaaaataa agttgtaaga gataaacccg cctatataaa ttcatatatt ttcctctccg 780
   ctttgaatac tgtataagag ctcattttta caacaattac caacaacaac aaacaacaaa 840
   caacattaca attacattta caattatcga tacaatggat ccgattcgtt ctcggacacc 900
   ttctcccgct cgagagctcc ttcctggacc acaaccagat ggagtgcaac caacagctga 960
   taggggtgta tctcctccag ccggtggacc tcttgacggc ttacctgcaa ggcgcacaat 1020
   gtcccgtacc agattgccca gtccaccagc accaagtcca gcgttttcag cgggcagctt 1080
   ctctgacctc ctgagacagt ttgatccctc tttgttcaac acctcactgt ttgactcact 1140
   tccgcctttt ggggctcacc atactgaagc cgctactggt gagtgggatg aggtgcagtc 1200
   aggcttaaga gcagctgatg ccccaccacc tacgatgagg gtggcagtaa ctgctgctag 1260
   gccaccgaga gctaaaccag ctccaagacg cagagcagca caaccttccg atgcatctcc 1320
   tgctgctcaa gtcgatcttc gcactttagg gtatagccag caacaacagg agaagatcaa 1380
   acctaaggtc cgaagtacag ttgcgcaaca ccatgaagcc cttgttggtc atgggttcac 1440
   tcatgcccac atagttgcac tatcccaaca tcctgctgct cttggaactg ttgcggtgaa 1500
   gtaccaggac atgattgctg ctttacctga agcaacacac gaggcaatag tcggtgttgg 1560
   caaacagtgg tctggcgcta gggctctaga agccctcctt acagttgcag gagaattgcg 1620
   gggacctccc ttgcagctcg ataccggaca attgctgaag attgccaaac gtggtggtgt 1680
   tacggcagta gaagcagtgc atgcttggag gaatgctcta actggagcac ccttgaatct 1740
   cacgccagaa caagtggtcg ctatcgcctc ccatgatggt ggaaaacaag cactagaaac 1800
   tgtccaaaga ttattgcctg ttctttgtca ggcacacgga cttaccccac aacaagtcgt 1860
   tgctatagcc tcccatgatg gagggaagca agcgttagaa acagtgcagc ggctactccc 1920
   tgtattatgc caggctcatg gtctaactcc acaacaagtg gtggctatag cctcacacga 1980
   tggtggtaaa caggcacttg aaaccgtcca aagactcctg ccggtcctct gccaggcaca 2040
   cggcctcacc cccgaacaag tggtggctat tgcttcgcat gatggaggta agcaggcttt 2100
   agagacagtc cagagactac tacccgttct atgccaggcc catggtttga ccccggaaca 2160
   ggttgttgct attgcgtcaa ataagggcgg caagcaagcg ttggaaaccg ttcaagcatt 2220
   actccctgtt ctctgtcaag cacatgggct aacgcccgag caggttgttg caattgcatc 2280
   acatgatgga ggaaagcagg ccttagaaac ggtacaggca cttttaccag tcctttgcca 2340
   agcacacggg cttacacccg aacaagtggt cgctattgca agtaatatag gtggaaaaca 2400
   agcactggaa accgtgcagg cgcttttgcc ggtattatgc caagctcacg gcctaactcc 2460
   tgaacaggtg gttgcgattg cctcaaatgg tgggggtaaa caggcactgg agactgtgca 2520
   gcggcttttg cctgttttgt gtcaagctca tggattgaca ccagagcagg tggtcgctat 2580
   agctagtaac attggaggta aacaagcgct tgaaaccgtg caacgtctgc tgccagttct 2640
   atgtcaagct catgggttga ccccacaaca ggttgtagcg atcgcttcca ataaaggagg 2700
   aaagcaagct ctagaaacgg tgcagaggct cctcccggtt ctttgtcagg cgcatggatt 2760
   gaccccggag caggtggtcg caatcgccag tcatgatgga ggtaagcagg ccttggaaac 2820
   cgttcaggcg ttactcccgg ttctatgcca ggcgcatggc ctgacccctg aacaggttgt 2880
   ggcgatagcc agtaacggcg ggggaaagca ggcacttgaa accgtacaac gactcctccc 2940
   agtcctttgt caagcccacg gattgactcc agaacaagta gttgctatag cttcgaataa 3000
   gggaggaaag caggcccttg aaacagttca gcgtcttttg ccagtgttgt gtcaagcaca 3060
   cggattgact cctgaacagg ttgtcgccat tgcatctaat atcggtggta agcaagctct 3120
   cgaaaccgta cagcgactct tgcctgttct atgccaagcg catggcttga cgccggaaca 3180
   ggtggtagcc atagcaagca acataggtgg caaacaagct cttgaaacag ttcaaaggtt 3240
   gttacctgtg ctttgccaag cccacggttt gacccctcaa caggtggttg ctatagcatc 3300
   acatgatggg ggacggcctg ctcttgagac agtgcagcgc ctgttgcccg tgttgtgtca 3360
   agcgcatggc ttaacaccgg aacaggtcgt ggcaattgcg tcaaatattg gcggcaaaca 3420
   agcgctggaa accgttcagc gactcttgcc tgttctgtgc caagctcacg gtctgacgcc 3480
   ccaacaggtt gttgccattg cttcaaatgg aggagggagg ccagcccttg agtcgattgt 3540
   cgcacagcta tctcggcccg accctgcttt agccgctctg acaaatgatc atcttgtggc 3600
   tctcgcctgc ttaggaggtc gcccagcttt agacgcagta aaaaagggtc tacctcatgc 3660
   tccggcctta atcaagagga cgaatcgtag aatcccagaa cgaacgagcc atcgcgtagc 3720
   cgatcacgct caagttgtta gggttttagg tttttttcag tgtcattcac atccggcaca 3780
   agctttcgat gatgccatga cccagtttgg tatgtcaagg catggattac tgcaactttt 3840
   cagaagagta ggagtgacag agctcgaagc cagaagcgga actctgccac ccgctagcca 3900
   aagatgggat aggatattgc aggcgagtgg aatgaagcgc gcgaaaccat ctccaacaag 3960
   cactcaaacc ccggatcaag cgagtttgca cgctttcgca gattctctcg aacgagattt 4020
   ggatgcccct tctccaatgc acgaaggtga tcaaactagg gcgagtagca ggaagaggtc    4080
   taggagtgat cgtgcagtta cgggcccctc agcacaacag tcttttgagg tcagggtgcc    4140
   agaacaaagg gacgctttac atctcccatt gtcttggcgt gtaaaaaggc cgcgaactag    4200
   tattggaggg ggattaccgg acccagggac ccccactgct gctgatctag ctgcttctag    4260
   tacggtaatg cgcgagcaag acgaggatcc atttgctggg gcagctgatg acttccccgc    4320
   attcaacgaa gaagaattag catggttgat ggagttactg ccacagtaag cttgtcctgc    4380
   tttaatgaga tatgcgagaa gcctatgatc gcatgatatt tgctttcaat tctgttgtgc    4440
   acgttgtaaa aaacctgagc atgtgtagct cagatcctta ccgccggttt cggttcattc    4500
   taatgaatat atcacccgtt actatcgtat ttttatgaat aatattctcc gttcaattta    4560
   ctgattgtac cctactactt atatgtacaa tattaaaatg aaaacaatat attgtgctga    4620
   ataggtttat agcgacatct atgatagagc gccacaataa caaacaattg cgttttatta    4680
   ttacaaatcc aattttaaaa aaagcggcag aaccggtcaa acctaaaaga ctgattacat    4740
   aaatcttatt caaatttcaa aagtgcccca ggggctagta tctacgacac accgagcggc    4800
   gaactaataa cgctcactga agggaactcc ggttccccgc cggcgcgcat gggtgagatt    4860
   ccttgaagtt gagtattggc cgtccgctct accgaaagtt acgggcacca ttcaacccgg    4920
   tccagcacgg cggccgggta accgacttgc tgccccgaga attatgcagc atttttttgg    4980
   tgtatgtggg ccccaaatga agtgcaggtc aaaccttgac agtgacgaca aatcgttggg    5040
   cgggtccagg gcgaattttg cgacaacatg tcgaggctca gcaggac                  5087

## Description

### FIELD OF THE INVENTION

The invention relates to a use of a kit or system for expressing two or more nucleic acid sequences of interest in a eukaryotic organism, cell or tissue of a eukaryotic organism, comprising a library of promoters and an effector that can bind to effector binding sites in the promoters of the libraries. The invention further relates to eukaryotic cells or eukaryotic organisms, notably plant cells or plants, comprising two or more nucleic acid constructs of interest, each nucleic acid construct of interest comprising a promoter selected from the library. The invention also relates to a process of expressing two or more nucleic acid sequences of interest in eukaryotic cells, in cells or tissue of a eukaryotic organism..

### BACKGROUND OF THE INVENTION

One of the goals of synthetic biology is to establish artificial transcriptional networks for the expression of novel signalling or metabolic pathways. While this was successfully done in unicellular microorganisms (Brophy and Voigt, 2014), the development of such transcriptional networks in higher eukaryotes, in particular in higher plants, presents additional difficulties due to the lack of sufficient available data, multicellularity and chromatin structure. One prerequisite for the construction of such networks is the availability of a range of transcriptional promoters of varying strengths that can be activated by orthogonal transcription factors. Since such promoters are not available in nature, they have to be designed, synthesized, assembled in functional transcription units and tested.

Several approaches have been adopted for promoter engineering, including random mutagenesis, saturation mutagenesis of poorly conserved regions and the engineering of hybrid promoters (Blazeck and Alper, 2013). An additional desirable property of synthetic promoters is their activation by orthogonal transcription factors (TFs). This is particularly relevant for multicellular organisms such as higher plants, for example if the aim is to express a biosynthesis pathway exclusively in one tissue or under specific conditions, such as biotic or abiotic stresses. Most promoter libraries described so far in yeast are based on upstream activating sequences (UAS) of endogenous TFs (Blazeck and Alper, 2013). If the TF on which the design of these synthetic promoters is based is expressed constitutively, or only in specific tissues or conditions, this will considerably restrict their potential use. For constitutive TF, this would prevent the activation in specific tissues, and conversely for tissue-specific TFs, this would restrict the use of the promoters to a specific tissue, preventing their general application for other tissues or conditions. A set of promoters whose expression can be induced by an orthogonal TF would circumvent these problems. In this case, the localization and pattern of expression would be determined by the orthogonal TF, which itself can be put under the control of a tissue-specific promoter for example.

Tornoe et al., Gene 297 (2002) 21-32 describes the generation of a synthetic mammalian promoter library by modification of sequences spacing transcription factor binding sites. Also discussed is inter alia that a theoretically unlimited number of genes can be expressed at constant individual levels by randomized promoters, and that basal promoters may be combined with sequences conferring e.g. growth-phase dependent inducibility, followed by randomization of promoter sequences.

Boch et al., Science (2009), vol. 326, no. 5959 describe TAL-Type III effectors. Geißler et al., PLOS ONE, (2011), vol. 6, no. 5, e19509 describe transcriptional activators of human genes with programmable DNA-specificity. Farzadfard et al., ACS SYNTHETIC BIOLOGY (2013), vol. 2, no. 10, 604-613 describe tunable and multifunctional eukaryotic transcription factors based on CRISPR/Cas. De Lange et al., THE PLANT JOURNAL, (2014), vol. 78, no. 5, 753-771 describe TAL effectors as tools for synthetic biology.

Departing from the prior art, it is an object of the invention to provide means for coordinated expression of more than one nucleic acid sequence of interest, notably in eukaryotic organisms or cells thereof. It is another object to provide means for orthogonal expression of nucleic acid sequences of interest at desired expression levels. It is a further object of the invention to provide building blocks to construct artificial transcriptional networks by introducing positive or negative feedback loops, or transcriptional cascades.

### SUMMARY OF THE INVENTION

For solving these objects, the invention provides the subject-matters of claims 1 to 13.

The present invention makes use of, in one embodiment, the recently discovered Transcription Activator-Like Effectors ("TALEs" or "TAL effectors") (Boch and Bonas, 2010, Boch et al., 2009). The modular structure of their DNA binding site allows the design of truly orthogonal transcription factors by selecting promoters of the invention having an effector binding site that does not occur in the genome, thus minimizing off-target gene activation. Since their discovery, TALEs have been used both for transcription activation and genome editing, the latter by fusing them to nucleases (de Lange et al., 2014). Tools for the construction of custom-designed TALEs are available (Weber et al., 2011).

The invention describes the design, construction and testing of a library of synthetic promoters that can be activated by effectors such as a designer TALE ("dTALE") transcription factor. Based on a remarkably simple architecture comprising the dTALE-DNA binding site, a TATA box and degenerate sequences flanking these constant regions on one or both sides, these promoters exhibit properties that make them ideal tools for the development of complex transcriptional networks or for metabolic engineering applications. First, as shown in the examples for the case of the GUS reporter gene, they exhibit a usable range of expression levels in transient assays that varies from 5 to 90% of that of the 35S promoter, which is one of the strongest plant promoters available. Large variations in expression levels of individual genes within a biosynthesis pathway have been observed in plants. For example, genes encoding the cytochrome P450 monooxygenases of the parthenolide pathway in feverfew (*Tanacetum parthenium*) can be expressed at levels several hundred fold higher than the gene encoding the germacrene A synthase, which represents the first committed step of the pathway (Liu et al., 2014). Thus, the range of expression levels delivered by the library of promoters is particularly useful to tune the expression of individual pathway genes in metabolic engineering projects. This can serve to optimize pathway flux but also to avoid excess production of certain enzymes, which may cause metabolic overload of the host cells. Second, in the absence of the effector, no or low background levels of expression were detected, demonstrating the orthogonal nature of the effector/promoter system.

The library of promoters described herein can be used for metabolic engineering of complex biosynthetic pathways requiring the simultaneous and tunable expression of multiple genes. This is particularly relevant in plants, where specific tissues or conditions can then be tested and compared for their performance in producing specific classes of compounds. Furthermore, these synthetic promoters provide the basis for the design and construction of complex regulatory networks that contain transcriptional cascades, positive and negative feedback loops. Thus, their potential reaches beyond the field of metabolic engineering.

### BRIEF DESCRIPTION OF THE DRAWINGS

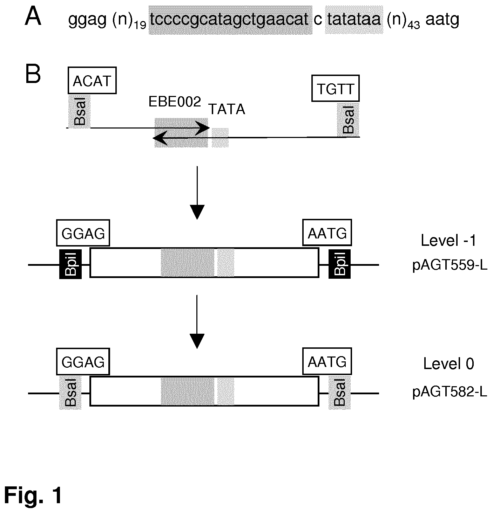

**Figure 1****. Design and cloning procedure of the STAPs (TALE-activated promoters).** A. general sequence of the STAPS. A constant dTALE binding site (EBE002) of 19 bases is highlighted by shading in blue and the TATA box in green-shaded. The atg triplet at the end of the promoter corresponds to the translation start codon. The degenerate sequences upstream and downstream of the constant region are indicated by (n)₁₉ and (n)₄₃ respectively. B. Cloning procedure of the STAPS. The library was synthesized using two degenerate oligonucleotides overlapping over the constant region and cloned into a level -1 Golden Gate vector. Subsequent cloning into a level 0 vector allowed elimination of aberrant products (for example dimers) and false positives. The level 0 vectors can then be used for assembly into transcription units.

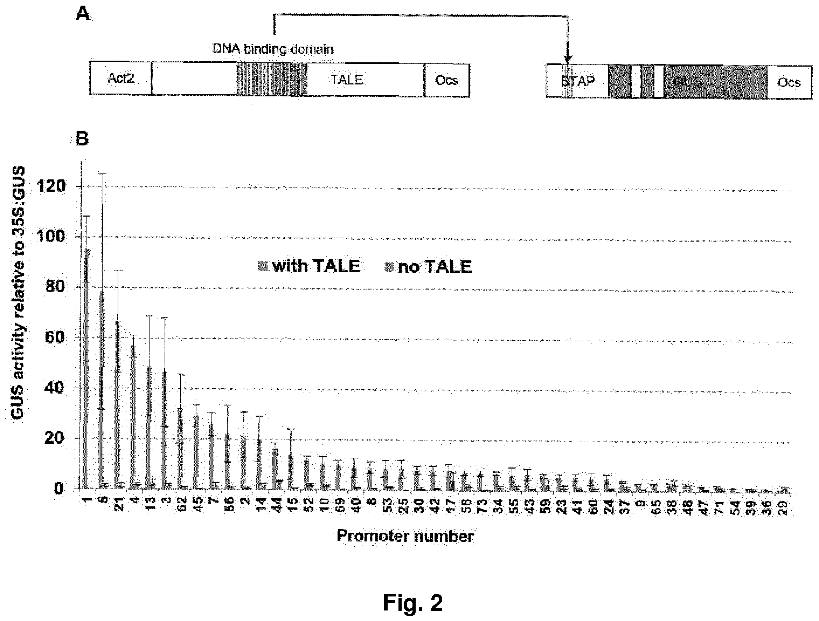

**Figure 2****. Testing the STAPs in a transient assay with a GUS reporter gene.** A. Overview of the constructs used for the transient assay. The dTALE which binds to the constant EBE002 sequence of the STAPs, is under the control of the constitutive and moderate *Act2* promoter. The repeats of the dTALE are indicated by narrow vertical lines and labeled "DNA binding domain". The STAPs control the expression of a GUS transgene with introns (represented by white bars within the shaded coding sequence) to prevent expression in *Agrobacterium* which is used for the infiltration of *N. benthamiana* leaves. B. Distribution of the GUS activity values for the STAPs with or without the dTALE, expressed in percentage of 35S:GUS expression.

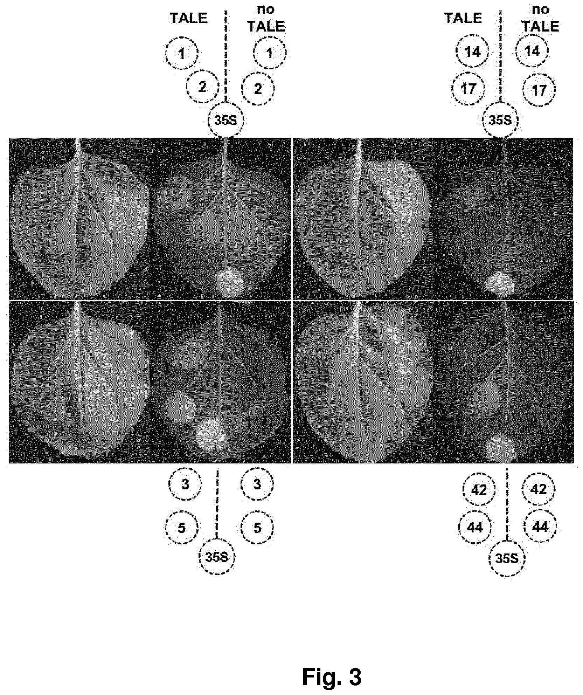

**Figure 3****. Testing selected STAPs with GFP.** Eight different STAPs displaying different levels of expression with the GUS reporter gene were tested with GFP. *N. benthamiana* leaves were infiltrated with *Agrobacterium* strains carrying different STAP:GFP constructs with (left side) or without (right side) the dTALE. Pictures of the leaves were taken in normal light (first and third column) or under UV illumination (second and fourth column) to visualize GFP.

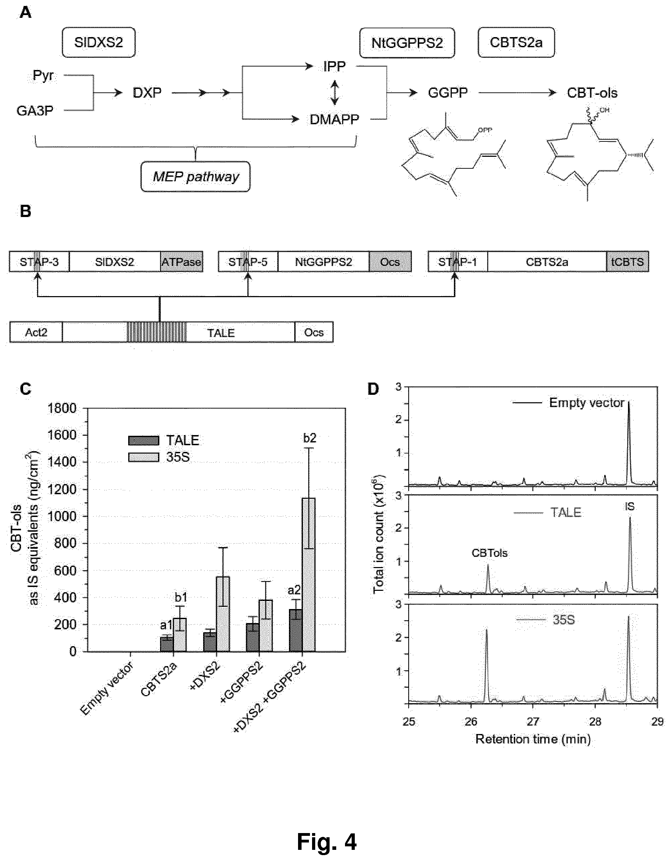

**Figure 4****. Using STAPs for metabolic engineering of a plant diterpene.** A. overview of the pathway for cembratrienols (CBTols) including the methylerythritol-phosphate (MEP) pathway, highlighting the genes that were over-expressed in *N. benthamiana.* Pyr, pyruvate; GA3P, glyceraldehyde-3-phosphate; DXP, 1-deoxyxylulose 5-phosphate; IPP, isopentenyl diphosphate; DMAPP, dimethylallyl diphosphate; GGPP, geranylgeranyl diphosphate; SIDXS2, tomato 1-deoxyxylulose 5-phosphate synthase; NtGGPPS2, tobacco GGPP synthase; CBTS2a, tobacco cembratrienol synthase. B. Overview of the constructs used for the overexpression of SIDXS2, NtGGPPS2 and CBTS2a using STAPs. The numbers of the STAPs correspond to the numbers in Figures 2 and 3. C. Comparison of TALE-driven expression and p35S-driven expression in *N. benthamiana* leaves agro-infiltrated with p19 and CBTS2a, DXS2 and GGPPS2 in different combinations. Two leaves in three individual plants were infiltrated for each construct combination (n=6). Mean CBTol amounts are expressed as equivalents of the internal standard (IS, sclareol) ± standard error. Groups indicated by different letters (a1/a2, and b1/b2) differ significantly from each other regarding their CBTol values (P<0.05, Student's t-test and two-way ANOVA). No CBTol was detected in leaves expressing the empty T-DNA vector as a control.

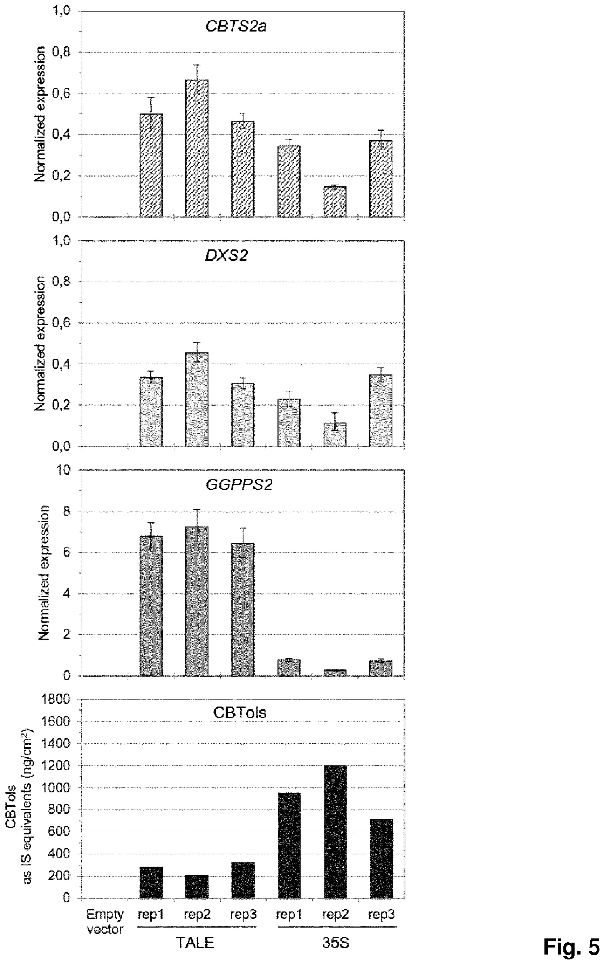

**Figure 5****.** TALE- and 35S-driven expression of the CBTS2a, DXS2 and GGPPS2 genes in agro-infiltrated N. benthamiana leaves. Transcript levels were quantified by RT-PCR in three selected biological replicates from samples used in Figure 4 (rep1-3) from agro-infiltrated leaves co-expressing p19, CBTS2a, DXS2 and GGPPS2 driven by STAPS (TALE) or the 35S promoter (35S). Mean expression values of three technical replicates ± SD and the corresponding CBTol amounts for each sample are given.

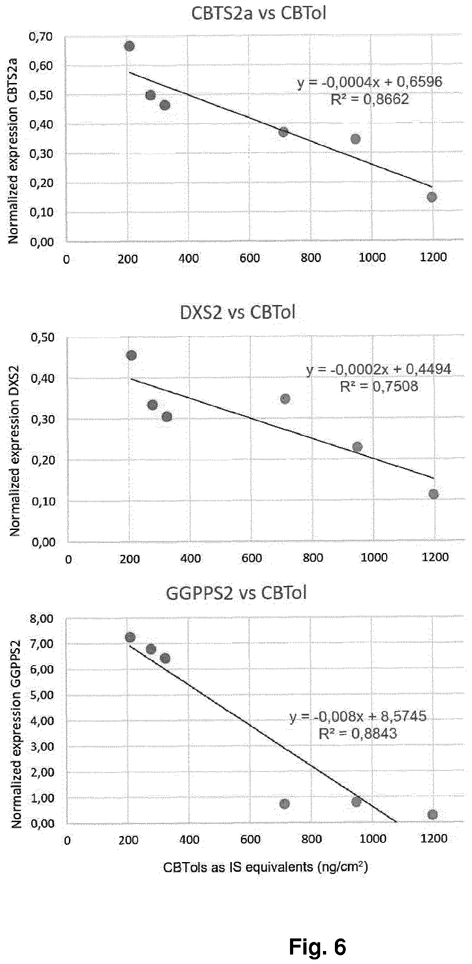

**Figure 6****.** Correlation between gene expression and CBTol level. Expression data from Figure 5 was plotted against CBTol levels. Trend lines and their corresponding parameters are shown. Red and green dots correspond to samples with the STAPs and 35S promoter respectively.

### DETAILED DESCRIPTION OF THE INVENTION

The library of multiple promoters is a set of unique multiple elements. In the library or set of multiple promoters used in the invention, each promoter comprises, in 5' to 3' direction, (i) an effector binding site comprising at least 10 contiguous bases, wherein the base sequences of effector binding sites of the multiple promoters have a sequence identity of at least 75%, (ii) a TATA box, and (iii) a downstream sequence segment comprising at least 20 contiguous bases. In an alternative embodiment, each promoter comprises, in 5' to 3' direction, (I) an upstream sequence segment of at least 10 contiguous based, (II) an effector binding site comprising at least 10 contiguous bases, wherein the base sequences of effector binding sites of the multiple promoters have a sequence identity of at least 75%, and (III) a TATA box. The promoters used in the invention are also referred to herein as "STAPS" which stands for "synthetic TALE-activated promoters". The TATA box is a known element of the core of eukaryotic promoters. The TATA box has the core DNA sequence 5'-TATAA-3' or a variant and may be followed by three or more A-T base pairs. The TATA box may be defined as comprising the sequence 5'-TATAW-3', preferably 5'-TATAWW-3', or 5'-TATAWWW-3', wherein W stands for either A or T. In the examples below, a TATA box of sequence 5'-TATATAA-3' is used.

The promoters of the library further have the effector binding site of item (i). The nucleic acid or base sequence of the effector binding site allows binding of effectors to the promoters such as effectors derived from TAL effectors, i.e. effectors having repeat units of a TAL effector. The effector binding site comprises at least 10 contiguous bases. Preferably, it comprises at least 15, more preferably at least 17 contiguous bases. The minimum length of the base sequence of the effector binding site may be chosen such that it is unique among promoter sequences of the eukaryotic organism or cell wherein the promoters are used for expressing nucleic acid sequences of interest. In this way, expression of a nucleic acid sequence of interest can be made orthogonal, i.e. not interfering with expression of endogenous genes. There is no strict upper limit for the length of the effector binding site. However, the length does not need to be longer than necessary for specific binding of an effector targeted to the effector binding site. Thus, the effector binding site generally has a length of 30 or less contiguous bases, preferably less than 25 contiguous bases, more preferably less than 20 contiguous bases. Preferred lengths of the effector binding site are from 10 to 25 contiguous bases, preferably 14 to 20 contiguous bases.

The effector binding sites of the multiple promoters of the library or set of promoters have the same or very similar base sequence over the length described above such that the same effector can bind to all of the multiple promoters of the library. For this purpose, the base sequences of effector binding sites of the multiple promoters have a sequence identity of at least 75%, preferably at least 85%, more preferably at least 90, more preferably at least 95%, and even more preferably of at least 98%. Any pair of two effector binding sites of the multiple promoters of the library may have the minimum sequence identities as defined in the previous sentence. This means that there is no effector binding site in the library having a smaller sequence identity as given in the penultimate sentence to an effector binding site of any other promoter in the library. In calculating sequence identities between two base sequences of effector binding sites, up to one gap is allowed, meaning that up to one gap can be neglected in the calculation. In a preferred embodiment, no gaps are allowed, meaning that no gap is neglected in the calculation. In one embodiment, the base sequences of the effector binding sites are identical among the promoters in a library.

Preferred embodiments of effector binding site are as follows: the effector binding site of the multiple promoters is a sequence segment of from 10 to 30 contiguous bases and the base sequences of effector binding sites of the multiple promoters have a sequence identity of at least 85%, preferably of at least 90%, more preferably at least 95% within such segment; the effector binding site of the multiple promoters is a sequence segment of from 13 to 27 contiguous bases and the base sequences of effector binding sites of the multiple promoters have a sequence identity of at least 85%, preferably of at least 90%, more preferably at least 95% within such segment; the effector binding site of the multiple promoters is a sequence segment of from 16 to 24 contiguous bases and the base sequences of effector binding sites of the multiple promoters have a sequence identity of at least 85%, preferably of at least 90%, more preferably at least 95% within such segment.

As an alternative to the definition of the high similarity of the effector binding sites among promoters of the library by way of percentage sequence identity, the minimum similarity of the effector binding sites may be defined as follows. In one embodiment, the effector binding sites of the promoters of the library comprise at least 10 contiguous bases and the base sequences of effector binding sites of the multiple promoters are the same over a length of 10 contiguous bases except that one or two bases, preferably one base, may be exchanged. Alternatively, one base may be deleted or added and one base may be exchanged.

In another embodiment, the base sequences of effector binding sites of the multiple promoters are the same over a length of 15 contiguous bases except that up to three bases, preferably up to two bases, more preferably up to one base, may be exchanged. Alternatively, one base may be deleted or added and one or two bases, preferably one base, may be exchanged.

In another embodiment, the base sequences of effector binding sites of the multiple promoters are the same over a length of 20 contiguous bases except that up to four bases, preferably up to three bases, more preferably up to two bases, more preferably one base, may be exchanged. Alternatively, one base may be deleted or added and up to three bases, preferably up to two bases, more preferably one base, may be exchanged.

Different libraries may be made wherein the base sequences of the effector binding sites have larger or more differences to those indicated above. However, within a given library, the base sequences of the effector binding sites of the multiple promoters are the same or very similar over the lengths as defined above such that the same effector can bind to all of the multiple promoters of the library. However, it is not necessary that a given effector binds to all effector binding sites of the promoters of the library with the same strength.

If the effector binding sites described above of a library of promoters are considered a first effector binding site, one or more promoters of the library may have a second or even a further effector binding site upstream of the TATA box for allowing binding of a second effector to the promoters. A first effector is capable of binding to the first effector binding site. A second (or further) effector is capable of binding to a second (or further) effector binding site. The first effector may be a transcription activator capable of binding to the first effector binding site. The second effector may also be a transcription activator or may be a transcription repressor binding to the second effector binding site. Generally, the properties of a second or further effector binding site in terms of length and minimum base sequence identities correspond to those given above for the first effector binding site. In the case of two or more effector binding sites, the two or more effector binding sites on a given promoter may have the same base sequence. Preferably, however, the two or more effector binding sites differ in nucleic acid sequence for allowing binding of different effectors. For this purpose, the base sequences of multiple effector binding sites on the same promoter generally have a sequence identity of at most 80%, preferably at most 60%. It is possible that all promoters of a given library have a second or a further effector binding site. In another embodiment, some but not all of the promoters of a given library have a second or further effector binding site.

The promoters of a library further comprise a sequence segment comprising at least 20 contiguous bases downstream from the TATA box. The multiple promoters differ in the downstream sequence segments, which constitutes the library used in the invention. The length of the downstream sequence segment is preferably at least 25, more preferably at least 30 (contiguous) nucleotides. There are no strict limits for the upper limit of the length of the downstream sequence segment. There is generally no advantage of having the downstream sequence segment longer than 150 contiguous nucleotides. Thus, the length is preferably 150 contiguous nucleotides or less, preferably 100 contiguous nucleotides or less. In one embodiment, there are at most 40 nucleotides in the downstream sequence segments of the library. Possible ranges of the length of the downstream sequence segments of the promoters of the library are as follows: from 25 to 150 bases, from 30 to 100 bases, from 40 to 90 bases, or from 40 to 70 bases. The promoters in the library may have downstream sequence segments of the same length, i.e. number of base pairs. However, it is not necessary that the number of base pairs is the same among different promoters of the library. The promoters of the library differ at least in the downstream sequence segments. The term "differ in the downstream sequence segments" means that the downstream sequence segments differ in length and/or in base sequence. Downstream sequence segments of different promoters of the library differ by at least 1, preferably by at least 2, more preferably by at least 5 bases over the downstream sequence segment lengths indicated above. Since there may be many promoters in the library, this still allows a wide variety of downstream sequence segments within a library.

In one embodiment, the promoters of the library have downstream sequence segments of identical lengths, and different promoters of the library differ by at least 1, preferably by at least 2, more preferably by at least 5 bases in the downstream sequence segments.

Promoters of the library of promoters may have an upstream sequence segment located upstream of said effector binding site(s), said upstream sequence segment comprising at least 10 contiguous nucleotides, wherein the multiple promoters differ, additionally or alternatively to the differences in the downstream sequence segments, in the upstream sequence segments. The length of the upstream sequence segment is preferably at least 20 nucleotides, more preferably at most 30 nucleotides. There is generally no advantage of having the upstream sequence segment longer than 100 contiguous nucleotides. Thus, the length is preferably 100 contiguous nucleotides or less, preferably 50 contiguous nucleotides or less, more preferably 30 contiguous nucleotides or less. The sequence variety provided by the optional upstream sequence segment provides for more additional variety among the library of promoters. The upstream sequence segments differ in length and/or in base sequence. The upstream sequence segments of different promoters may differ by at least 1, preferably by at least 2, more preferably by at least 4 bases. Since there may be many promoters in the library, this allows a wide variety of upstream sequence segments within a library. In one embodiment, the promoters of the library have upstream sequence segments of identical lengths, and different promoters of the library differ by at least 1, preferably by at least 2, more preferably by at least 4 bases. In a preferred embodiment, the promoters of the library have upstream sequence segments of identical lengths and downstream sequence segments of identical lengths, wherein the possible lengths are as defined above for the upstream and downstream sequence segments.

The promoters of the library may have a sequence stretch in between the effector binding site and the TATA box. The effector binding sites may be separated from the TATA box by a nucleic acid sequence segment of at most 500, preferably at most 300, more preferably of at most 100 bases, and even more preferably at most 50 or even at most 20 bases. However, it is also possible that there is no or a very short sequence stretch in between the effector binding site and the TATA box, such as one nucleotide as in the examples below. Among members of the library, it is not necessary that they all have a stretch in between the effector binding site and the TATA box of the same number of nucleotides.

The promoters of the library may have a sequence stretch in between the TATA box and the downstream sequence segments. In one embodiment, the downstream sequence segments follow directly the TATA box, wherein the TATA box is taken to end with the last A or T base following, in 5' to 3' direction, the sequence TATA of the TATA box. This means that differences between downstream sequence segments are determined starting with the base following the last A or T base of the TATA box.

A library of promoters used in the invention comprises multiple promoters, i.e. at least 2 promoters. A library preferably comprises at least 3, more preferably at least 5, even more preferably at least 10 promoters, even more preferred at least 20 promoters differing in the base sequence of said downstream sequence segment and optionally of said upstream sequence segment.

Some or all promoters of the library may comprise or encode a 5'-untranslated region (5'-UTR) and/or a start codon downstream of said downstream sequence segment. Downstream sequence segments and a 5'-UTR may or may not overlap. An example of a commonly used 5'-untranslated region is the Omega-enhancer of the Tobacco Mosaic Virus (Gallie et al., Nucleic Acids Res. 1987 Apr 24;15(8):3257-73.).

The library used in the invention may be produced by *de novo* synthesis of the promoters by nucleic acid synthesis methods. Another approach for synthesis of the library is described in the examples and illustrated in Fig. 1. In this approach, overlapping degenerate oligonucleotides are synthesized that overlap in the region of the effector binding sites. The degeneracy of the oligonucleotide introduces the variability into the downstream and, optionally, the upstream sequence segment. The sequence variation of the downstream sequence segment of the promoters and, optionally, the upstream sequence segment is provided by portions of the oligonucleotides that do not overlap. Mixtures of forward and reverse oligonucleotides are cloned into an acceptor vector, preferably by cloning making use of type IIs restriction sites such as by "Golden Gate Cloning". Clones obtained after transformation in bacteria may be selected and the promoters may be sequenced.

In general, the library used in the invention is stored in plasmids such as the level -1 or level 0 vectors depicted in Fig. 1. The plasmids or vectors generally contain a selectable marker and may be maintained in bacteria. The library may be in a form wherein the elements of the library are stored in separate containers. This allows, after having identified a suitable promoter, to identify the specific container containing the identified promoter. In another embodiment, the multiple promoters of the library are present in one container, i.e. are mixed. In such embodiment, the library can still be screened and clones containing a selected promoter may be identified using generally known cloning and sequencing methods (see examples below).

Herein, nucleic acid (or base) sequences are given in 5' to 3' direction and refer to the coding strand unless specified differently. The meaning of "coding strand" is as defined in Figure 11.1 in GENES IX, Lewin, Benjamin, Jones and Bartlett Publishers. Lengths of base or nucleotide sequences are given in numbers of contiguous bases. The same numerical values apply where the lengths are given with regard to base pairs or nucleotides.

The invention also provides a use of a kit or system comprising (A) a library of multiple promoters according to any of the embodiments described herein and (B) an effector capable of binding to the effector binding sites of all members of the library. Alternatively, a nucleic acid molecule encoding the effector is used as item (B) of the system or kit. In a first embodiment (further described below), the effector comprises a repeat domain comprising repeat units derived from or of a transcription activator-like (TAL) effector, said effector being capable of binding to the (first) effector binding site of the promoters. In a second embodiment (further described below), the effector comprises an RNA (such as gRNA) having complementarity to the effector binding site of the promoters, whereby the effector may further comprise an engineered CRISPR/Cas9-transcription factor (TF)-fusion.

The library of item (A) of the kit or system may be present in one container, i.e. in a mixed state. Alternatively, the library may be present in the kit or system such that each element (promoter) of the library is contained in a separate contained. In effector or a nucleic acid molecule encoding it or a component thereof is generally contained in a container separate from containers containing elements of the library. Information given herein on the library and the effector also relate to the library and effector, respectively, of the kit, system, organisms, cells and processes.

A main application of the invention is the expression of two or more, preferably three or more, more preferably four or more, nucleic acid sequences of interest in a eukaryotic organism or cell or tissue thereof. Preferably, the expression of the two or more nucleic acid sequences of interest is coordinated in the sense that the expression levels of the two or more nucleic acid sequences of interest are predetermined by selection of different appropriate promoters from a library of promoters for achieving desired expression levels for each of the two or more nucleic acid sequences of interest. Further, expression may be orthogonal in that it is independent from endogenous transcription factors in the host cells or organism. For this purpose, the at least two nucleic acid sequences of interest may be placed under the control of different promoters from the library of promoters to obtain a nucleic acid construct of interest for each of the nucleic acid sequences of interest.

For expression of two or more nucleic acid sequences of interest in a eukaryotic organism or cell or tissue thereof, the invention provides a eukaryotic cell or organism excluding human beings and a process of expressing two or more nucleic acid sequences of interest in eukaryotic cells, in cells or tissue of a eukaryotic organism. If two nucleic acid sequences of interest are to be expressed, promoters from the library of promoters are used for the nucleic acid sequence of interest that differ in their downstream sequence segments and/or the upstream sequence segments. If three or more nucleic acid sequences of interest are to be expressed, the promoters used for at least two of the three or more nucleic acid sequences of interest differ in their downstream sequence segments and/or the upstream sequence segments. It is also possible that all three or more nucleic acid sequences of interest are expressed using promoters from the library that differ in their downstream sequence segments and/or their upstream sequence segments.

For expressing two or more nucleic acid sequences of interest, nucleic acid constructs of interest may be prepared, wherein each nucleic acid construct of interest comprises a promoter as described herein and, downstream thereof, a nucleic acid sequence of interest to be expressed. Thus, also described is a set of two or more nucleic acid constructs of interest, each construct comprising a different nucleic acid sequence of interest. At least two of said two or more nucleic acid constructs of interest have promoters that differ in the base sequence of their downstream sequence segments and/or of the upstream sequence segments. A nucleic acid construct of interest may further comprise other regulatory elements for expressing the nucleic acid sequences of interest, such as a terminator downstream of the nucleic acid sequences of interest. The use of terminators for eukaryotic gene expression is part of the general knowledge of the skilled person.

The eukaryotic cell, tissue or organisms of the invention may be produced by introducing the two or more nucleic acid constructs of interest simultaneously, e.g. by co-transformation, or consecutively. The nucleic acid constructs of interest used for transformation or transfection are generally part of a transformation vector that contains a selectable marker that can be used for selecting transformed cells. For production of stably transformed organisms or cells, selection procedures are performed for selecting cells or organisms having the nucleic acid constructs stably introduced into the genome of the cells or organism. For transient expression of the nucleic acid sequences of interest, selecting transformed cells or organisms is generally not done. In the process of expressing two or more nucleic acid sequences of interest in eukaryotic cells, in cells or tissue of a eukaryotic organism, or in a eukaryotic organism, eukaryotic cells, tissue of a eukaryotic organism, or a eukaryotic organism may be provided with the two or more nucleic acid constructs of interest as described above. Information given herein on elements of the promoters also applies to the promoters used in the eukaryotic cell or organism and to the promoters used in the expression process.

Methods of transforming or transfecting eukaryotic cells or organisms are part of the general knowledge.

The effector used in the invention, or a component thereof, is capable of binding to the effector binding sites and of activating transcription from the promoters. The effector used in the invention may be an effector that does not naturally occur in the eukaryotic organism, or cells thereof, where the nucleic acid sequences of interest are to be expressed. Accordingly, the effector binding sites of the promoters may be binding sites that are not recognised by endogenous effectors such as transcription factors or repressors present in the eukaryotic organism, or cells thereof, where the nucleic acid sequences of interest are to be expressed. The eukaryotic cell or organism further comprises or is provided with an effector-expressing nucleic acid construct encoding an effector, wherein the effector can bind to the effector binding sites of the promoters and activate transcription from said promoters. Activation of expression from one or more nucleic acid construct(s) of interest may be combined with repression of expression from of one or more further nucleic acid construct(s) of interest.

In a first embodiment of effectors usable in the invention, the effector is based on the recently discovered Transcription Activator-Like Effectors (TALEs) (Boch and Bonas, 2010, Boch et al., 2009, WO 2012/104729). TALEs are virulence factors of plant pathogens from the genus *Xanthomonas* that are translocated via a type III secretion system inside the plant cell. The TALEs are then imported into the nucleus, where they bind to specific DNA sequences and transcriptionally activate gene expression. DNA binding is mediated by a central repetitive region, formed by, generally, up to 33 tandem repeats of a 33 to 35 amino acid residue motif, each repeat corresponding to one DNA base pair of the target sequence. The amino acid sequences of the repeats are nearly identical, except amino acid positions 12 and 13, the so-called repeat variable diresidues (RVD). Repeats with different RVDs show different DNA base pair preferences, and consecutive RVDs in a TALE correspond directly to the DNA sequence in the binding side, resulting in a simple one-repeat-to-one-base pair code (Weber et al., 2011). The tandem repeats are also referred to as "modules" or "units" herein. The modular structure of the DNA binding site of TALEs allows the design of orthogonal transcription factors by selecting a DNA sequence that does not occur in the genome, thus minimizing off-target gene activation. Natural TAL effectors such as the well-studied AvrBs3 are monomeric proteins comprising an N-terminal domain that contains a translocation signal of the secretion system, a central repeat domain containing the tandem repeats, and a C-terminal domain containing a nuclear localization signal (NLS) and an activation domain (AD). The central repeat domain is sufficient for binding to target DNA.

In the first embodiment of the effectors for use in the invention, the effector comprises a repeat domain comprising repeat units (or "modules") derived from a TAL effector. The effector comprises the desired number of modules, whereby the number of modules in the effector may correspond to the number of contiguous bases (or base pairs) in the effector binding sites of the promoters. Thus, the number of repeats is preferably at least 10, more preferably at least 15. More generally, at least 7 repeats are necessary for activation of transcription. That the repeat units or modules are derived from a transcription activator-like (TAL) effector means that the amino acid sequence of each repeat corresponds closely to the natural amino acid sequence of the repeats except for the RVDs at positions 12 and 13 of the repeats. The nucleotide sequence of the avrBs3 gene encoding a TAL effector is given, in a codon-optimized form for expression in *Nicotiana tabacum*, in the supporting information of (Weber et al., 2011). The sequence stretches of the repeats are identified therein. The repeats localized N-terminally in the effector correspond to the 5' end of the effector binding site, the C-terminal repeats correspond to the 3' end of the effector binding site.

The repeats may have the following consensus sequence (see Figure 2 of Boch, J. and Bonas, U. (2010) in the one-letter code of amino acid residues: where x stands for any amino acid residue at positions 12 and 13 which determine base recognition specificity and constitute the repeat variable diresidue (RVD). Thus, the repeats may have the amino acid sequence of SEQ ID NO:51 except that the amino acid residue at position 4 may, alternatively to E, be D or A, the amino acid residue at position 11 may, alternatively to S, be N, and the amino acid residue at position 32 of SEQ ID NO. 51 may, alternatively to A, be D. The term "derived from a TAL effector" means that the repeat units or modules of the repeat domain may comprise an amino acid sequence as defined in the previous sentence.

As is known in the art (Boch and Bonas, 2010, Boch et al., 2009, WO 2012/104729), the amino acid sequence of the RVD of the effector corresponds to base pairs in the effector binding site as follows:
HD for recognition of C/A,
NA for recognition of A/C/G/T
ND for recognition of C
NI for recognition of A/G,
NG for recognition of T/A,
NN for recognition of G/A,
NH for recognition of G
NV for recognition of A/G/C
N* for recognition of C/G,
HG for recognition of T/C
HN for recognition of G/A
HI for recognition of A
S* for recognition of A/C/G/T
wherein * represents absence of amino acid 13 at the RVD, and the repeat units in the repeat domain of the effector are combined in an order corresponding to base sequence in the effector binding domain. Comprehensive interrogation of natural TALE DNA-binding modules and transcriptional repressor domains can be found in Cong, L et al. NATURE COMMUNICATIONS, Volume 3, article number 968.

The effector may also contain parts of the N-terminal domain of a TAL effector. However, the N-terminal translocation signal for the type III translocation system is generally not required, unless this translocation system is employed for providing cells with the effector. The effector generally contains a transcription activation domain at the C-terminal end. In this case, the effector acts as an activator of expression from the promoter. As a C-terminal activator (or activation) domain, that of the AvrBs3 gene may be used, a nucleotide sequence of which is given, in a codon-optimized form for expression in *Nicotiana tabacum*, in the supporting information of (Weber et al., 2011). Activator domains of other TAL effectors may also be used. Further, activator domains of other transcriptional activators may be used, such as the transcriptional activator domain of Virion protein 16 (VP16) of herpes simplex virus type 1 (Cress et al., Science 4 January 1991:Vol. 251 no. 4989 pp. 87-90) or of yeast Gal4 (Ma et al., Cell, Volume 48, Issue 5, 13 March 1987, Pages 847-853). Alternatively, the activator domain may be replaced by a repressor domain, whereby the effector may be used for repressing transcription from the promoter. Transforming effectors into transcriptional repressors was successfully done in various eukaryotic organisms including plants (Blount et al., 2012, Mahfouz et al., 2012). Examples of transcriptional repressor domains include the plant SRDX domain (Hiratsu et al., 2003, Plant Journal, 34:733-739) and the human SID and KRAB domains (le Cong et al., 2012, Nature Communications, doi:10.1038/ncomms1962). Weber et al., 2011 describes methods for producing TALE-based effectors, vectors encoding them and their use.

There are several possible ways of providing eukaryotic cells, tissue of a eukaryotic organism, or a eukaryotic organism with the effector. In one example, a nucleic acid construct expressibly encoding the effector (also referred to herein as "effector-expressing nucleic acid construct") is generated. In such construct, the encoded effector is under the expression control of a promoter that may or may not be a promoter from the library of the library of multiple promoters. In a basic embodiment, the promoter of the effector-expressing nucleic acid construct is not a promoter from the library of promoters described herein, but may still be a regulated or tissue-specific promoter. In such embodiment, expression of multiple nucleic acid sequences of interest can be controlled by controlling expression of the effector. Alternatively, the promoter is a constitutive promoter in the cells or organism used for expression. The effector-expressing nucleic acid construct may then be provided into the cells or into cells of the organism such that it can be expressed therein.

In another example, the effector is introduced into said cells, into cells of said tissue, or into cells of said eukaryotic organism as a protein. Proteins may be transformed into cells from cell-free compositions as described in WO 2004/046360. Specific examples are injecting a solution containing the effector protein in tissue of an organism. For this purpose, the effector may be fused with a membrane translocation sequence enabling the direct introduction of the effector protein into cells.

In a further example, the effector is introduced into cells of a eukaryotic organism or into a eukaryotic organism by crossing a eukaryotic organism containing the nucleic acid constructs of interest integrated into the genome, such as the nuclear genome, with a eukaryotic organism of the same species containing in its genome an effector-expressing nucleic acid constructs encoding the effector as described above. Preferably, the parent organisms contain the respective nucleic acid constructs homozygously such that all progeny contains a copy of the desired constructs.

In a second embodiment of the effectors, effectors based on the CRISPR/Cas9 system are used (Fineran and Dy, 2014, Hsu et al., 2014, van der Oost et al., 2014). The CRISPR/Cas9 enzyme is a nuclease whose sequence specificity is determined by a guide RNA (gRNA) complementary to a target sequence (van der Oost et al., 2014). As such, CRISPR/Cas9 is suited for genome editing, with the possibility to target several loci by co-expressing or even concatenating multiple gRNAs, which can be cloned easily due to their small size (Hsu et al., 2014). In this regard, CRISPR/Cas9 genome editing is simpler to implement than TALE-based editing, because for each locus to be targeted, two new dTALEs need to be designed and expressed (de Lange et al., 2014). The CRISPR/Cas system was also modified to generate TFs (CRISPR/Cas-TF), whose specificity is determined by gRNAs (Nissim et al., 2014; WO 2014/197748 and WO 2015/006747). To achieve this, the gRNAs were expressed by RNA pol II promoters, requiring the editing of the RNA by the Csy4 enzyme (Qi et al., 2012). The fact that the specificity of the CRISPR/Cas-TF is determined at the RNA level, and not at the protein level as with the TALEs, constitutes a limitation for the design of more complex regulatory networks with activating or repressing steps. Because all CRISPR/Cas proteins will recognize the gRNAs and their target DNA regardless of whether they are modified to be transcriptional activators or repressors, co-expression of both types of CRISPR/Cas-TFs in a single cell could result in unpredictable expression of the target genes. Also, a single CRISPR/Cas-TF protein controlling the whole network cannot act simultaneously as a repressor and activator on different targets. Thus, to introduce a negative regulatory step in a CRISPR/Cas regulatory network, Nissim et al. had recourse to a miRNA (Nissim et al., 2014). In contrast, distinct dTALEs with either repressor or activating properties can be co-expressed, thus allowing more flexibility and possibilities for the design of regulatory networks (de Lange et al., 2014).

In this second embodiment, the effector comprises at least two components. The effector comprises an RNA having complementarity to an effector binding site of the promoters. The effector preferably comprises a guide RNA (gRNA), more preferably a single-guide RNA (sgRNA), and a CRISPR/Cas-transcription factor-fusion. The sgRNA is an engineered functional fusion of crRNA and tracrRNA (Jinek et al., 2012). The eukaryotic cell or organism comprises or is provided with a nucleic acid construct encoding the gRNA or sgRNA. The eukaryotic cell or organism further comprises or is provided with a nucleic acid construct encoding a CRISPR/Cas-TF. The CRISPR/Cas is preferably CRISPR/Cas9.

The CRISPR/Cas9-TF is a fusion protein that comprises a domain of an engineered CRISPR/Cas9 (referred to as dCas9 in the prior art such as in Maeder, ML. Et al., 2013) protein that is engineered so as to be catalytically inactive, and a domain of a TF. Examples of catalytically inactive engineered Cas9 proteins are given in WO 2015/006747. The TF can be a repressor or an activator. Fusions of dCas9 to TF domains have been used to activate reporter genes in E. coli and human cells (Bikard, D., 2013; Gilbert LA., 2013). WO 2014/197748 and WO 2015/006747 describe examples of CRISPR/Cas9-TF-fusions. Methods of expressing constructs encoding CRISPR/Cas9-effectors are known in the prior art cited herein. The TF is preferably heterologous to the eukaryotic cells or organism. An example of a CRISPR/Cas9-TF fusion is a dCas9-VP64 transcriptional activation domain fusion protein described by Maeder, ML. Et al., 2013. The dCas9-VP64 transcriptional activation domain fusion protein can be directed by single or multiple gRNAs to increase expression of specific endogenous human genes.

The nucleic acid constructs encoding the effector-RNA and the CRISPR-/Cas-TF-fusion are under transcriptional control of promoters in the constructs for co-expressing them in the eukaryotic cells or the organism. When the sgRNA is transcribed in the cells, the sgRNA directs sequence-specific binding of a CRISPR/Cas9/gRNA complex to the target sequence, i.e. an effector binding site of a promoter used in the invention. The complex comprises the CRISPR/Cas9-TF fusion complexed with the gRNA or sgRNA that can hybridize to the effector binding site.

The eukaryotic organisms, or cells or tissue thereof, considered in the invention for expression may be plants, animals, or fungi. Among these, plants are preferred. Among animals, mammals such as rodents are preferred, but humans are excluded from protection. However, the invention may be used for expressing proteins in human (and other animal) cells. Among fungi, yeasts are preferred.

In a basic embodiment, the effector acts as activator of expression on nucleic acid constructs of interests containing promoters as described herein. Thus, when expression of the effector in eukaryotic cells starts, or when a directly introduced effector occurs in cell nuclei, the effector can bind to the effector binding sites of nucleic acid constructs of interest and activate expression of the nucleic acid sequences of interest contained in the constructs.

The promoters used in the invention can be used for metabolic engineering of complex biosynthetic pathways, including simultaneous and tunable expression of multiple genes. This is particularly relevant in plants, where specific tissues or conditions can be tested and compared for performance in producing specific classes of compounds. Furthermore, the promoters provide the basis for the design and construction of complex regulatory networks that contain transcriptional cascades, positive and negative feedback loops. Thus, their potential reaches beyond the field of metabolic engineering. Positive feedback can simply be done by adding to one or more of the promoters an effector binding site for a second effector. Negative feedback can be achieved similarly by replacing the activator domain of a second effector by a repressor domain. Transforming effectors into transcriptional repressors was successfully done in various eukaryotic organisms including plants (Blount et al., 2012, Mahfouz et al., 2012). Thus, the library of promoters provide the starting material for designing complex regulatory circuits that could include transcriptional cascades, Boolean operators, as well as positive and negative feedback loops.

Thus, in the eukaryotic cell, tissue or organism, and in the process of expressing two or more nucleic acid sequences of interest, one or more of said nucleic acid constructs of interest may have a second or a further effector binding site upstream of the TATA box. The second or a further effector binding site was described above in the context of the library. The second or further effector binding site may be located between the first effector binding site and the TATA box or, preferably, is/are located upstream of the first effector binding site. One or more of the nucleic acid constructs of interest may contain a nucleic acid of interest encoding a second effector capable of binding to the second or further effector binding site of one or more other nucleic acid constructs of interest for activating (where the second effector is or contains an activator) or repressing (where the second effector is or contains a repressor) transcription of nucleic acid sequences of interest in one or more other nucleic acid constructs of interest containing the second or further effector binding site.

Given the ever-increasing number of genome sequences that become available, including from higher plants, it is possible to design effectors that do not or minimally interfere with endogenous gene expression and, conversely, that are not bound by endogenous TFs. Thus, the effector/promoter combinations used in the invention can be made a truly orthogonal system that neither depends nor interferes with endogenous transcription activation networks. The relevance of this system for metabolic engineering is high. Our experiments on transient expression assay for diterpene production in *N. benthamiana* demonstrated that several genes can be co-expressed by the effector/promoter-system at levels that are comparable to the strong CaMV 35S promoter. While using the same promoter to co-express multiple genes in a transient assay works properly, in stable transgenic plants this can lead to undesirable consequences such as gene silencing (Peremarti et al., 2010). Therefore, a particularly relevant application of these promoters will be in stable transgenic plants, where coordinated tissue-specific expression of pathway genes can be done without requiring the identification of multiple endogenous promoters conferring the same expression profile and with different expression levels. Hence, by simply changing the promoter driving the expression of the activating effector and keeping the rest of the construct of interest identical, it will be possible with minimum cloning effort to test different tissues in parallel for performance in producing a compound of interest. For example, one could compare tissues such as the seed endosperm, the epidermis, glandular trichomes, or root hairs, for which specific promoters are available (Potenza et al., 2004, Tissier, 2012).

Accordingly, also described is a process of screening multiple promoters from a library of multiple promoters for expressing a given nucleic acid sequence of interest in a pre-selected eukaryotic organism or cells thereof, comprising
preparing multiple nucleic acid constructs of interest, each containing the same nucleic acid sequence of interest and, upstream and operably linked thereto, different promoters from the library of promoters as defined in item 1,
introducing the multiple nucleic acid constructs of interest in the pre-selected eukaryotic organism or cells thereof,
following expression from each of said multiple nucleic acid constructs of interest in the pre-selected eukaryotic organism or cells thereof, and
selecting the promoter from the nucleic acid construct of interest showing suitable or desired expression of said nucleic acid sequence of interest in the pre-selected eukaryotic organism or cells thereof.

The process steps of such screening process may be performed based on general knowledge of the skilled person and the teaching given herein. Once suitable promoter(s) is (are) identified, it can be used for expressing the nucleic acid sequence of interest using the construct containing the selected promoter.

### EXAMPLES

In the following Examples, the design and construction of libraries of synthetic TALE-activated promoters (STAPs) are described. These were assembled in Golden Gate compatible vectors to facilitate their downstream use (Engler et al., 2014). These STAPs were evaluated with reporter genes (GUS and GFP) in transient assays in *Nicotiana benthamiana,* then, tested in a metabolic engineering set-up for the production of a plant diterpene.

### Construction of a library of synthetic TALE-activated promoters (STAPs)

To generate a library of promoter sequences as described in Figure 1, two degenerate primers, Talpro7 and Talpro8 (see sequences below), containing the TALE-DNA binding site (EBE-002 or dTALE-2 binding site, described in (Weber et al., 2011)) and degenerate sequences on either side of the DNA binding site were made and annealed at a construct pL2-dTALE-2 (Weber et al., 2011) with the difference that the 35S promoter in pL2-dTALE-2 was replaced by the Arabidopsis Act2 promoter in pICH74043; the nucleotide sequence of pICH74043 is presented in SEQ ID NO: 52) and a 35S:GFP construct as internal reference (construct pAGM4731, (Engler et al., 2014)). As control, the pAGT615-n constructs were also infiltrated without the TALE. A construct with 35S:GUS (pICH75181, (Engler et al., 2014)) co-infiltrated with 35S:GFP was used as a positive control and external reference. We infiltrated the reporter constructs with and without the TALE and a positive control (35S:GUS) on different sectors of the same leaf to eliminate leaf-to-leaf variation. Agrobacterium strains were grown in LB medium, resuspended in infiltration medium (10 mM MES, 10 mM MgSO₄) at a final OD₆₀₀ of 0.3 for infiltration. The plants were then placed back in the greenhouse for five days before analysis. For quantitative GUS assays, leaf discs of 0.9 cm diameter within the infiltrated areas were excised and transferred to microtubes containing three 0.2 mm steel beads and immediately frozen in liquid N₂. Frozen leaf tissues were then homogenized twice using a Retsch mixer mill MM400 at a frequency of 30 Hz for 1 min. The leaf powder was then resuspended in 1 mL phosphate buffered saline (pH 7.4), vortexed and filtered through a 0.2 µM PTFE filter on a 96-well plate (Chromafil, Macherey-Nagel, Germany). GUS activity was measured using the fluorogenic 4-methylumbelliferyl β-D-glucopyranoside (MUG) substrate using published procedures at 10, 30 and 60 min (Jefferson et al., 1987). In parallel, 200 µL of the extracts was used to measure GFP fluorescence. The GFP fluorescence of each sample was used as normalization factor. The strength of the individual promoters was then expressed as a percentage of the expression with the 35S promoter, based on the control from the same leaf.

### GFP assays

Selected promoters from the pAGT582-n series were cloned into a level 1 vector (pICH47732) in front of GFP (vector pICH41531, (Engler et al., 2014)) and the ocs terminator (pICH41432). The constructs were introduced into Agrobacterium GV3101(pMP90) and expressed in *N. benthamiana* with or without the TALE-EBE002 (see above). The 35S:GFP construct (pAGM4731, see above) was used as positive control. The plants were incubated for three to five days and photos were taken under a UV lamp.

### Diterpene metabolic engineering constructs

The coding sequences of the *SIDXS2* (pAGT345), *NtGGPPS2* (pAGT169) and *CBTS2a* (pAGT238) genes were those described in (Brückner and Tissier, 2013). They were cloned behind the promoters from pAGT582-3, pAGT582-5 and pAGT582-1 respectively. The terminators used were SI vATPase1 (pICH 71431), Ocs (pICH 41432), and CBTS (pAGT168) respectively (Brückner and Tissier, 2013, Engler et al., 2014). These fragments were assembled into level 1 vectors pICH47751, pICH47742 and pICH47802 respectively. The vectors were transformed into *Agrobacterium tumefaciens* GV3101:pMP90 and used for *N. benthamiana* leaf infiltration as described above. The corresponding constructs with the 35S promoter were as described in (Brückner and Tissier, 2013). Metabolite extraction and diterpene GC-MS measurements were performed as described in (Brückner and Tissier, 2013) with the following modifications. The 1 mL hexane extracts from the 6 leaf discs per sample were not evaporated and 160µL of these extracts were combined with 40µL of internal standard (IS) (50µM Sclareol stock solution in hexane), mixed, centrifuged and transferred to a GC-vial. Samples were measured on a QP2010 Ultra GC-MS system (Shimadzu) in the following conditions. 1 µL was injected splitless at 250°C with a 1 min sampling time in a RXi-5Sil MS 30 m, 0.25 mm diameter column (Restek). Elution was done with a constant He flow of 0.9 mL/min. The oven temperature was raised from 50°C to 300°C (7°C/min), then to 320°C (50°C/min) and he Id for 2 min. The interface and the ion source temperatures were 300°C and 250°C respective ly and the ionization voltage was set at 70 V.

### Quantitative gene expression analysis in infiltrated leaf tissue

Total RNA was extracted from infiltrated leaf discs from which diterpenes were extracted by hexane wash before. Leaf discs were homogenized to a fine powder using a Mixer Mill MM-400 (Retsch) and RNA was extracted with the Spectrum™ Plant Total RNA Kit (Sigma-Aldrich) according to the manufacturer's specifications. Any genomic contamination was removed before cDNA synthesis using the DNA-free Kit (Ambion) according to manufacturer instructions. The first strand of cDNA was synthesized from 1 µg total RNA using Maxima H Minus First Strand cDNA Synthesis Kit (Thermo Scientific) and a mix of oligo(dT)18 and random hexamer primers. The cDNA was diluted 10-fold and 1 µl was used in a 10 µl reaction mix containing 1 µl (2 pmol/µl) of both forward and reverse primers and 2 µl my-Budget 5x EvaGreen QPCR Mix II (Bio&Sell). Quantitative real-time PCR was performed in 96-well plates using the CFX Connect real-time PCR detection system (Bio-Rad). The thermal profile of the reaction was as follows: an initial denaturation at 95°C for 15 min, followed by 40 cycles at 95°C for 15 seconds and 58°C for 30 seconds with fluorescence acquisition after each cycle. To verify primer specificity, a dissociation curve was finally generated by increasing the temperature from 65 to 95°C. qPCR primers for the amplification of *CBTS2a*, *DXS2* and *GGPPS2* genes were designed using the Primer3 program (Untergrasser et al., 2012). The *N. benthamiana* eukaryotic elongation factor 1a was selected as endogenous reference for normalization of transcript levels, and PCR amplified using primers as described in Liu et al., 2012. To check the specificity of the *CBTS2a*, *DXS2* and *GGPPS2* primers, qPCR was performed also on *N. benthamiana* cDNA and no amplification could be detected in any of the control samples (Figure 5). The sequence of the oligonucleotides used for real-time PCR are provided in the supplemental data. All the cDNA samples were amplified in three technical replicates from the same RNA preparation and the mean value was considered. Expression of transgenes in infiltrated leaf tissue was calculated using the 2^{(delta-Ct)} method (Livak and Schmittgen, 2001).

### Results

### Design and construction of a library of synthetic TALE-activated promoters

The structure of the STAPs is presented in Figure 1A. They contain an eighteen base-long DNA binding domain, named EBE002, flanked by a nineteen base long degenerate sequence immediately upstream, and a consensus TATA box downstream. The TATA box is followed by a forty-three base long degenerate sequence and the ATG start codon. This design is based on the observation that the DNA-binding sequence of TALEs from bacterial pathogens that activate genes *in planta* are typically located close to the transcription start site (less than 100 bp) and that insertion of a DNA binding sequence at position -55 or -40 was sufficient to confer TALE-mediated inducibility (Kay et al., 2009). The library was cloned using the MoClo system based on Golden Gate cloning (Werner et al., 2012). The scheme for the construction of the library of STAPs is presented in Figure 1B. The library was made in a level -1 vector of the MoClo system, yielding pAGT559-L, and transferred in a level 0 vector, yielding pAGT582-L. Level 0 vectors contain parts (typically promoter, cDNA, terminator) which are brought together in level 1 vectors to assemble functional transcription units. Transferring the library from level -1 to level 0 ensures that only fragments containing correct overhangs are kept, thus limiting the number of false positives and increasing the efficiency of cloning in subsequent steps. Approximately 50 clones from the level 0 library were picked and checked for the presence of a fragment of the expected size. Fourty-three clones were thus selected and sequenced (see sequence listing). All promoter sequences contained the EBE002 DNA binding site.

### Transient assays of the STAPs with a GUS reporter gene

Each STAP was cloned upstream of the GUS reporter gene and the ocs transcription terminator in a level 1 vector, which can also be used for *Agrobacterium tumefaciens* T-DNA transfer (Figure 2A). The transcriptional activity of these promoters was first evaluated in transient assays in *Nicotiana benthamiana* by measuring GUS activity in protein extracts. The dTALE is under control of the Act2 promoter (Figure 2A), which provides sufficient expression for the activation of transcription. For the quantification of the expression, leaves were infiltrated in triplicate with the STAP:GUS constructs, with or without the dTALE and with a 35S:GFP construct. GFP fluorescence was measured and used as a normalization factor. The results (Figure 2B) show a distribution of expression strengths ranging from less than 1% to over 90% of that of the 35S promoter. Since for some promoters there is significant residual background expression without the TALE, the useful range of expression in this transient assays is estimated to be between 5 to 90% of that of the 35S promoter. For most STAPs, however, no significant expression was detected in the absence of the TALE, indicating that the activity seen with the TALE does not stem from endogenous activators, establishing the orthogonality of the promoters.

### Transient assays of selected STAPs with GFP

To confirm these results with another reporter gene, a selection of eight STAPs with different expression strengths (STAPs numbers 1, 2, 3, 5, 14, 17, 42 and 44) were cloned in front of the GFP gene and transiently expressed in *N. benthamiana.* The results (Figure 3) are consistent with those of the GUS assay, indicating that the strength of expression for GUS and GFP seems to be independent of the transcribed gene. For all the tested STAPs, no GFP signal can be seen in the absence of the TALE, confirming the high-specificity of the synthetic promoters. The strongest promoters (numbers 1, 3 and 5) show a signal intensity approaching that of the 35S promoter, while the weaker promoters (numbers 17 and 42) still produce a detectable signal.

### Using the STAPs for diterpene metabolic engineering in N. benthamiana

We next sought to validate the utility of these promoters by using them for metabolic engineering of a plant diterpenoid. As output for our metabolic engineering assay we used the cembratrienol synthase (CBTS2a) from tobacco (Ennajdaoui et al., 2010, Wang and Wagner, 2003). This diterpene synthase produces a mix of two stereoisomers (α and β) of the macrocyclic cembratrienol (CBTol) (Figure 4). In plants, the terpenoid precursors isopentenyl diphosphate (IPP) and dimethylallyl diphosphate (DMAPP) are produced by two distinct pathways, the plastidic 2-C-methyl-D-erythritol-4-phosphate (MEP) pathway, and the cytosolic mevalonate (MEV) pathway (Vranova et al., 2012). Diterpenes are synthesized in the plastids and therefore predominantly use IPP and DMAPP from the MEP pathway. We previously showed that co-expression of 1-deoxyxylulose 5-phosphate synthase (DXS), the first step of the MEP pathway, with a geranylgeranyl diphosphate synthase (GGPPS) leads to more than a doubling of diterpene production in *N. benthamiana* transient assays (Brückner and Tissier, 2013). These assays were carried out with the strong Cauliflower Mosaic Virus 35S promoter controlling the expression of all the genes. The *DXS2* (tomato), GGPPS2 (tobacco) and the *CBTS2a* genes were cloned downstream of some of the strongest STAPs characterized above and transiently expressed together with the dTALE. The results show that both with the 35S promoter and the STAPs, the amount of CBTols produced is significantly higher when all genes *(CBTS2a, GGPPS2* and *DXS2*) are co-expressed compared to the other combinations *(CBTS2a* alone, *CBTS2a* + *DXS2* or *CBTS2a* + *GGPPS2*) (Figure 4C), confirming previous results with the 35S promoter (Brückner and Tissier, 2013). However, CBTols produced with the STAPs are around three-fold lower than with 35S. One explanation could be that gene expression is lower, leading to reduced CBTols levels. To check this, we measured transcript levels by quantitative RT-PCR from the same samples used for CBTol quantification. Surprisingly, the transcript levels were higher with the STAPs than with the 35S promoter (1.9 times for *CBTS2a,* 11.5 for *GGPPS2* and 1.5 for *DXS2*) (Figure 5). Thus, the lower CBTol levels cannot be caused by reduced transcript levels of the genes. Plotting the gene transcript levels versus the amount of CBTols produced indicates a negative correlation, regardless of the promoters used (STAPs or 35S) (supplemental Figure 1). Thus, this inverse effect of gene expression on metabolite levels does not seem to be specific to the STAPs.

These results show that the STAPs can be used for metabolic engineering applications and can deliver expression levels, which are comparable to those of the strongest known promoters.

### Discussion

We have validated the utility of the STAPs for metabolic engineering in a transient assay. We have confirmed that co-expression of GGPPS2 and DXS2 with the diterpene synthase CBTS2a leads to increased levels of CBTols (Figure 4C). However, the levels were lower than with the 35S promoter, which could have been due to lower gene expression levels. Unexpectedly, however, transcript levels measured by quantitative RT-PCR show that the STAPs confer significantly higher expression than the 35S promoter, in particular up to more than tenfold higher for the *GGPPS2* gene. Several hypotheses can be proposed to account for this negative correlation between transcript levels and product yield. The high expression of the enzymes could lead to negative feedback on the MEP pathway and therefore reduce flux through this pathway. Another possibility is that CBTols are produced in larger amounts with the STAPs but are modified by endogenous *N*. *benthamiana* enzymes (e.g. acyl- or glycosyltransferases, glutathione transferases), as has been shown for mono- and sesquiterpenoids (Ting et al., 2013). Here, however, one would have to invoke that the more CBTols are produced, the more they are modified in proportion. Yet another possibility is that the higher transcript levels are not accompanied by higher translation rates, rather in the contrary this leads to reduced translation activity. Testing these different hypotheses will require extensive additional experiments which should be carried out in the future.

Eukaryotic promoters are currently described as containing two major domains, namely the core promoter element, which is defined as the region where the transcription machinery is recruited and initiates transcription, and the upstream elements, which are bound by transcription factors that can either activate or repress transcription (Hahn and Young, 2011). It is well established that binding to specific upstream elements by transcription factors triggers the recruitment of the core transcription machinery and thereby activates transcription (Hahn and Young, 2011). Hardly anything is known, however, on how the sequence of the core promoter element influences transcriptional activity (Lubliner et al., 2013). In an early study, the analysis of 95 yeast promoters lead to the identification of a so-called "locator" signal 30-10 bp upstream of the transcription start site (TSS), which is rich in Ts and poor in As, and is "stronger" in promoters with high activity (Maicas and Friesen, 1990). More recently, a survey of 859 yeast core promoters identified T-richness upstream of the TSS as a strong predictor of promoter activity, regardless of whether the promoters are constitutive or inducible (Lubliner et al., 2013). As far as we could tell from the available literature, nothing is known in plants. Because of their short sequence and the presence of an identical DNA binding site, the STAPs constitute an appropriate material to study the influence of the base composition of the core promoter on transcription activity. Furthermore, it is possible that the sequence of the gene can have an impact on gene expression levels.

Our design of the STAPs was based on reports that identify the presence of AvrBs3-binding sites within 100 bp of the transcription start site of genes activated by AvrBs3, a TAL-effector from the plant pathogenic bacterium *Xanthomonas campestris* (Kay et al., 2007, Kay et al., 2009, Romer et al., 2007). Furthermore, Kay and collaborators showed that a TATA box immediately downstream of the TALE binding site leads to greatly increased expression (Kay et al., 2009). Although our design gave satisfactory results, further variations could provide additional regulatory options. For example, adding multiple TALE-binding sites could lead to increased expression, as shown in mammalian cells (Perez-Pinera et al., 2013). However, since our strongest expressors are almost on a par with the strong viral 35S promoter this may be of limited interest. More relevant would be the insertion of TALE-binding sites for positive or negative feedback regulation.

### Nucleic acid sequences used and referred to herein

Sequences of the promoters. The 19 base long DNA binding site of the TALE is indicated in bold letters:

Sequences of the primers used for quantitative gene expression analysis:
SEQ ID NO: 43 NsCBTS2a-1106F, 5'- CGATCCAAAGATGGGACGA-3',
SEQ ID NO: 44 NsCBTS2a-1257R, 5'- CATCTCTTTTTTCGCATAGTAGATGT-3',
SEQ ID NO: 45 NtGGPPS2-85F, 5'- CCTCCACAGAGACATTCTTATAGTTTC-3',
SEQ ID NO: 46 NtGGPPS2-227R, 5'- TCAAAGTCAAACTTAGGCAAGATG-3',
SEQ ID NO: 47 SIDXS2-556F, 5'- AGCATCTCCGCTGGTCTT-3',
SEQ ID NO: 48 SIDXS2-699R, 5'- CGAGTCAAGGAATCCTGCAT-3'
SEQ ID NO: 49: Talpro7: 5'-ttt ggtctc a acat GGAG (n)₁₉ tccccgcatagctgaacatc
SEQ ID NO: 50: Talpro8: 5'-ttt ggtctc a acaa CATT (n)₄₃ ttatata g atgttcagctatgcgggg
SEQ ID NO: 51: TAL effector repeat consensus
SEQ ID NO: 52: pICH74043 sequence including the Act2 promoter and untranslated sequence, the TAL effector coding sequence (underlined) and the Ocs terminator

### References

Bikard D, et al. Programmable repression and activation of bacterial gene expression using an engineered CRISPR-Cas system. Nucleic Acids Res. 2013.
Blazeck, J. and Alper, H.S. (2013) Promoter engineering: recent advances in controlling transcription at the most fundamental level. Biotechnol J, 8, 46-58.
Blount, B.A., Weenink, T., Vasylechko, S. and Ellis, T. (2012) Rational diversification of a promoter providing fine-tuned expression and orthogonal regulation for synthetic biology. PLoS One, 7, e33279.
Boch, J. and Bonas, U. (2010) Xanthomonas AvrBs3 Family-Type III Effectors: Discovery and Function. Annu. Rev. Phytopathol., 48, 419-436.
Boch, J., Scholze, H., Schornack, S., Landgraf, A., Hahn, S., Kay, S., Lahaye, T., Nickstadt, A. and Bonas, U. (2009) Breaking the code of DNA binding specificity of TAL-type III effectors. Science, 326, 1509-1512.
Brophy, J.A.N. and Voigt, C.A. (2014) Principles of genetic circuit design. Nat. Methods, 11, 508-520.
Brückner, K. and Tissier, A. (2013) High-level diterpene production by transient expression in Nicotiana benthamiana. Plant Methods, 9, 46.
de Lange, O., Binder, A. and Lahaye, T. (2014) From dead leaf, to new life: TAL effectors as tools for synthetic biology. The Plant Journal, 78, 753-771.
Engler, C., Kandzia, R. and Marillonnet, S. (2008) A one pot, one step, precision cloning method with high throughput capability. PLoS One, 3, e3647.
Engler, C., Youles, M., Gruetzner, R., Ehnert, T.M., Werner, S., Jones, J.D., Patron, N.J. and Marillonnet, S. (2014) A Golden Gate Modular Cloning Toolbox for Plants. ACS synthetic biology, **Epub ahead of print.**
Ennajdaoui, H., Vachon, G., Giacalone, C., Besse, I., Sallaud, C., Herzog, M. and Tissier, A. (2010) Trichome specific expression of the tobacco (Nicotiana sylvestris) cembratrien-ol synthase genes is controlled by both activating and repressing cis-regions. Plant Mol. Biol., 73, 673-685.
Fineran, P.C. and Dy, R.L. (2014) Gene regulation by engineered CRISPR-Cas systems. Curr. Opin. Microbiol., 18, 83-89.
Gilbert LA, et al. CRISPR-Mediated Modular RNA-Guided Regulation of Transcription in Eukaryotes. Cell. 2013.
Hahn, S. and Young, E.T. (2011) Transcriptional Regulation in Saccharomyces cerevisiae: Transcription Factor Regulation and Function, Mechanisms of Initiation, and Roles of Activators and Coactivators. Genetics, 189, 705-736.
Hsu, P.D., Lander, E.S. and Zhang, F. (2014) Development and Applications of CRISPR-Cas9 for Genome Engineering. Cell, 157, 1262-1278.
Jefferson, R.A., Kavanagh, T.A. and Bevan, M.W. (1987) GUS fusions: beta-glucuronidase as a sensitive and versatile gene fusion marker in higher plants. Embo j, 6, 3901-3907.
Jinek, M. et al., (2012) Science 337, 816-821.
Kay, S., Hahn, S., Marois, E., Hause, G. and Bonas, U. (2007) A bacterial effector acts as a plant transcription factor and induces a cell size regulator. Science, 318, 648-651.
Kay, S., Hahn, S., Marois, E., Wieduwild, R. and Bonas, U. (2009) Detailed analysis of the DNA recognition motifs of the Xanthomonas type III effectors AvrBs3 and AvrBs3Δrep16. The Plant Journal, 59, 859-871.
Koncz, C. and Schell, J. (1986) The Promoter of TI-DNA Gene 5 Controls the Tissue-Specific Expression of Chimeric Genes Carried by a Novel Type of Agrobacterium Binary Vector. Mol. Gen. Genet., 204, 383-396.
Liu, D., Shi, L., Han, C., Yu, J., Li, D., Zhang, Y. (2012) Validation of reference genes for gene expression studies in virus-infected Nicotiana benthamiana using quantitative real-time PCR. PLoS One, 7, e46451.
Liu, Q., Manzano, D., Tanic, N., Pesic, M., Bankovic, J., Pateraki, I., Ricard, L., Ferrer, A., de Vos, R., van de Krol, S. and Bouwmeester, H. (2014) Elucidation and in planta reconstitution of the parthenolide biosynthetic pathway. Metab Eng, 23, 145-153.
Livak, K. J., Schmittgen, T. D. (2001) Analysis of relative gene expression data using real-time quantitative PCR and the 2(-Delta Delta C(T)) Method. Methods, 25, 402-408.
Lubliner, S., Keren, L. and Segal, E. (2013) Sequence features of yeast and human core promoters that are predictive of maximal promoter activity. Nucleic Acids Res., 41, 5569-5581.
Mahfouz, M., Li, L., Piatek, M., Fang, X., Mansour, H., Bangarusamy, D. and Zhu, J.-K. (2012) Targeted transcriptional repression using a chimeric TALE-SRDX repressor protein. Plant Mol. Biol., 78, 311-321.
Maicas, E. and Friesen, J.D. (1990) A sequence pattern that occurs at the transcription initiation region of yeast RNA polymerase II Promoters. Nucleic Acids Res., 18, 3387-3393.
Nissim, L., Perli, S.D., Fridkin, A., Perez-Pinera, P. and Lu, T.K. (2014) Multiplexed and Programmable Regulation of Gene Networks with an Integrated RNA and CRISPR/Cas Toolkit in Human Cells. Mol. Cell, 54, 698-710.
Peremarti, A., Twyman, R.M., Gomez-Galera, S., Naqvi, S., Farre, G., Sabalza, M., Miralpeix, B., Dashevskaya, S., Yuan, D.W., Ramessar, K., Christou, P., Zhu, C.F., Bassie, L. and Capell, T. (2010) Promoter diversity in multigene transformation. Plant Mol. Biol., 73, 363-378.
Perez-Pinera, P., Ousterout, D.G., Brunger, J.M., Farin, A.M., Glass, K.A., Guilak, F., Crawford, G.E., Hartemink, A.J. and Gersbach, C.A. (2013) Synergistic and tunable human gene activation by combinations of synthetic transcription factors. Nat Meth, 10, 239-242.
Potenza, C., Aleman, L. and Sengupta-Gopalan, C. (2004) Targeting transgene expression in research, agricultural, and environmental applications: Promoters used in plant transformation. In Vitro Cell Dev-PI, 40, 1-22.
Qi, L., Haurwitz, R.E., Shao, W.J., Doudna, J.A. and Arkin, A.P. (2012) RNA processing enables predictable programming of gene expression. Nat. Biotechnol., 30, 1002-1006.
Romer, P., Hahn, S., Jordan, T., Strauss, T., Bonas, U. and Lahaye, T. (2007) Plant pathogen recognition mediated by promoter activation of the pepper Bs3 resistance gene. Science, 318, 645-648.
Ting, H.M., Wang, B., Rydén, A.M., Woittiez, L., van Herpen, T., Verstappen, F.W., Ruyter-Spira, C., Beekwilder, J., Bouwmeester, H.J. and van der Krol, A. (2013) The metabolite chemotype of Nicotiana benthamiana transiently expressing artemisinin biosynthetic pathway genes is a function of CYP71AV1 type and relative gene dosage. New Phytol., 199, 352-366.
Tissier, A. (2012) Trichome specific expression: promoters and their applications. In Transgenic Plants, advances and limitations (Çiftçi, Y.O. ed: InTech, pp. 353-378.
Untergrasser, A., Cutcutache, I., Koressaar, T., Ye, J., Faircloth, B.C., Remm, M., Rozen, S.G. (2012) Primer3 - new capabilities and interfaces. Nucleic Acids Research, 50, e115.
van der Oost, J., Westra, E.R., Jackson, R.N. and Wiedenheft, B. (2014) Unravelling the structural and mechanistic basis of CRISPR-Cas systems. Nature Reviews Microbiology, 12, 479-492.
Vranova, E., Coman, D. and Gruissem, W. (2012) Structure and Dynamics of the Isoprenoid Pathway Network. Mol. Plant., 5, 318-333.
Wang, E. and Wagner, G.J. (2003) Elucidation of the functions of genes central to diterpene metabolism in tobacco trichomes using posttranscriptional gene silencing. Planta, 216, 686-691.
Weber, E., Gruetzner, R., Werner, S., Engler, C. and Marillonnet, S. (2011) Assembly of Designer TAL Effectors by Golden Gate Cloning. PLoS One, 6, e19722.
Werner, S., Engler, C., Weber, E., Gruetzner, R. and Marillonnet, S. (2012) Fast track assembly of multigene constructs using Golden Gate cloning and the MoClo system. Bioeng Bugs, 3, 38-43.

## Claims

1. A process of expressing two or more nucleic acid sequences of interest in eukaryotic cells, in cells or tissue of a eukaryotic organism, or in a eukaryotic organism excluding humans, comprising:
(a) providing eukaryotic cells, tissue of a eukaryotic organism, or a eukaryotic organism excluding humans comprising two or more nucleic acid constructs of interest, each nucleic acid construct comprising a promoter and, downstream thereof, a nucleic acid sequence of interest to be expressed, wherein each promoter of said nucleic acid constructs of interest is selected from a library of multiple promoters each comprising, in 5' to 3' direction,
(i) an effector binding site comprising at least 10 contiguous bases, wherein the base sequences of effector binding sites of the multiple promoters have a sequence identity of at least 75%,
(ii) a TATA box, and
(iii) a downstream sequence segment comprising at least 20 contiguous bases, wherein the downstream sequence segments of at least two nucleic acid constructs of interest differ, and
(b) providing the eukaryotic cells, tissue of a eukaryotic organism, or a eukaryotic organism of the previous step with an effector capable of binding to the effector binding sites of the promoters and activating transcription from said promoters.

2. The process according to claim 1, wherein step (b) comprises:
transfecting the eukaryotic cells, the tissue of a eukaryotic organism, or the eukaryotic organism obtained in step (a) with an effector-expressing nucleic acid constructs encoding said effector; or
introducing said effector into said cells, into cells of said tissue, or into cells of said eukaryotic organism obtained in step (a).

3. Eukaryotic cell or organism excluding humans, comprising two or more nucleic acid constructs of interest, each nucleic acid construct of interest comprising a promoter and, downstream thereof, a nucleic acid sequence of interest to be expressed, each construct comprising a different nucleic sequence of interest, wherein the promoters of said nucleic acid constructs of interest are selected from a library of multiple promoters each comprising, in 5' to 3' direction,
(i) an effector binding site comprising at least 10 contiguous bases, wherein the base sequences of effector binding sites of the multiple promoters have a sequence identity of at least 75%,
(ii) a TATA box, and
(iii) a downstream sequence segment comprising at least 20 contiguous bases, provided that at least two of said two or more nucleic acid constructs of interest have promoters that differ in their downstream sequence segments,
further comprising (an) effector-expressing nucleic acid construct(s) encoding an effector, wherein said effector can bind to the effector binding sites of the promoters of said two or more nucleic acid constructs of interest and activate transcription from said promoters.

4. The eukaryotic cell or organism according to claim 3, wherein said effector comprises a repeat domain comprising repeat units derived from a transcription activator-like (TAL) effector.

5. The eukaryotic cell or organism according to claim 3, wherein said effector comprises an RNA having complementarity to an effector binding site of said promoters, and said eukaryotic cell or organism comprises a nucleic acid construct encoding said RNA.

6. The eukaryotic cell or organism according to claim 5, wherein said effector comprises a guide RNA (gRNA) having complementarity to an effector binding site, preferably said effector-RNA is a single guide RNA (sgRNA) comprising a crRNA and a tracrRNA.

7. The eukaryotic cell or organism according to any one of claims 3 to 6, wherein one or more of said nucleic acid constructs of interest comprises a second or a further effector binding site upstream of the TATA box.

8. The eukaryotic cell or organism according to any one of claims 3 to 7, wherein one or more of said nucleic acid constructs of interest contains a nucleic acid construct of interest encoding an effector capable of binding to a second or further effector binding site for activating or repressing transcription of a nucleic acid sequences of interest in (a) nucleic acid construct(s) containing the second or further effector binding site.

9. Use of a system or kit for expressing two or more nucleic acid sequences of interest in a eukaryotic organism excluding humans or cell or tissue of a eukaryotic organism and for metabolic engineering of a biosynthetic pathway requiring simultaneous and tunable expression of multiple genes, comprising:
(A) a library of multiple promoters, each of said multiple promoters comprising, in 5' to 3' direction,
(i) an effector binding site comprising at least 10 contiguous bases, wherein the base sequences of effector binding sites of the multiple promoters have a sequence identity of at least 75%,
(ii) a TATA box, and
(iii) a downstream sequence segment comprising at least 20 contiguous bases,
wherein the multiple promoters differ in the downstream sequence segments, and
(B) an effector being capable of binding to the effector binding sites of said multiple promoters; or a nucleic acid molecule encoding said effector.

10. The use according to claim 9, wherein the effector binding site of the multiple promoters is a sequence segment of from 10 to 30 contiguous bases, preferably of from 13 to 27 contiguous bases, more preferably of from 16 to 24 contiguous bases; and/or
wherein the base sequences of effector binding sites of the multiple promoters have a sequence identity of at least 85%, preferably of at least 90%, more preferably at least 95%.

11. Use of a system or kit for expressing two or more nucleic acid sequences of interest in a eukaryotic organism excluding humans or cell or tissue of a eukaryotic organism, wherein the expression is coordinated in that the expression levels of the two or more nucleic acid sequences of interest are predetermined by selection of different promoters from a library of promoters for achieving desired expression levels for each of the two or more nucleic acid sequences of interest, comprising:
(A) a library of multiple promoters, each of said multiple promoters comprising, in 5' to 3' direction,
(i) an effector binding site comprising at least 10 contiguous bases,
(ii) a TATA box, and
(iii) a downstream sequence segment comprising at least 20 contiguous bases,
wherein the multiple promoters differ in the downstream sequence segments; and
wherein the base sequences of effector binding sites of the multiple promoters are the same over a length of
10 contiguous bases except that one or two bases may be exchanged, or one base may be deleted or added and one base may be exchanged, or
15 contiguous bases except that up to three bases may be exchanged, or one base may be deleted or added and one or two bases may be exchanged, or
20 contiguous bases except that up to four bases may be exchanged, or one base may be deleted or added and up to three bases may be exchanged and
(B) an effector being capable of binding to the effector binding sites of said multiple promoters; or a nucleic acid molecule encoding said effector.

12. The use according to any one of claims 9 to 11, said library of promoters further comprising an upstream sequence segment located upstream of said effector binding site, said upstream sequence segment comprising at least 10 contiguous bases, wherein said multiple promoters may differ in the said upstream sequence segment.

13. The use according to any one of claims 9 to 12, wherein said library is present in one container, or each promoter of said library is contained in a separate container.

## Patentansprüche

1. Verfahren zur Expression zweier oder mehrerer gewünschter Nukleinsäuresequenzen in eukaryotischen Zellen, in Zellen oder Gewebe eines eukaryotischen Organismus oder in einem eukaryotischen Organismus, wobei Menschen ausgeschlossen sind, umfassend:
(a) Bereitstellen von eukaryotischen Zellen, von Gewebe eines eukaryotischen Organismus oder eines eukaryotischen Organismus, wobei Menschen ausgeschlossen sind, die zwei oder mehr gewünschte Nukleinsäurekonstrukte umfassen, wobei jedes Nukleinsäurekonstrukt einen Promotor und strangabwärts davon, eine gewünschte zu exprimierende Nukleinsäuresequenz umfasst, wobei jeder Promotor des gewünschten Nukleinsäurekonstrukts ausgewählt ist aus einer Bibliothek zahlreicher Promotoren, die jeweils in 5'- nach 3'-Richtung folgendes umfassen:
(i) eine Bindungsstelle für einen Effektor, die wenigstens 10 zusammenhängende Basen umfasst, wobei die Basensequenzen der Bindungsstellen für den Effektor der zahlreichen Promotoren eine Sequenzidentität von wenigstens 75% aufweisen,
(ii) eine TATA-Box und
(iii) ein strangabwärts gelegenes Sequenzsegment, das wenigstens 20 zusammenhängende Basen umfasst, wobei sich die strangabwärts gelegenen Sequenzsegmente von wenigstens zwei gewünschten Nukleinsäurekonstrukten unterscheiden, und
(b) Bereitstellen von eukaryotischen Zellen, von Gewebe eines eukaryotischen Organismus oder eines eukaryotischen Organismus des vorhergehenden Schritts mit einem Effektor, der an die Bindungsstellen für den Effektor der Promotoren binden kann, und die Transkription ausgehend von den Promotoren aktivieren kann.

2. Verfahren nach Anspruch 1, wobei Schritt (b) folgendes umfasst:
Transfizieren eukaryotischer Zellen, des Gewebes eines eukaryotischen Organismus oder des eukaryotischen Organismus, die in Schritt (a) erhalten wurden, mit einem Effektor-exprimierenden Nukleinsäurekonstrukt, das für den Effektor codiert; oder
Einführen des Effektors in die Zellen, in die Zellen des Gewebes oder in die Zellen des eukaryotischen Organismus, die in Schritt (a) erhalten wurden.

3. Eukaryotische Zelle oder Organismus, wobei Menschen ausgeschlossen sind, umfassend zwei oder mehr gewünschte Nukleinsäurekonstrukte, wobei jedes gewünschte Nukleinsäurekonstrukt einen Promotor und strangabwärts davon, eine gewünschte zu exprimierende Nukleinsäuresequenz umfasst, wobei jedes Konstrukt eine unterschiedliche gewünschte Nukleinsäuresequenz umfasst, wobei die Promotoren der gewünschten Nukleinsäurekonstrukte ausgewählt sind aus einer Bibliothek zahlreicher Promotoren, die jeweils in 5'- nach 3'-Richtung folgendes umfassen:
(i) eine Bindungsstelle für einen Effektor, die wenigstens 10 zusammenhängende Basen umfasst, wobei die Basensequenzen der Bindungsstellen für den Effektor der zahlreichen Promotoren eine Sequenzidentität von wenigstens 75% aufweisen,
(ii) eine TATA-Box und
(iii) ein strangabwärts gelegenes Sequenzsegment, das wenigstens 20 zusammenhängende Basen umfasst, mit der Maßgabe, dass wenigstens zwei der zwei oder mehr gewünschten Nukleinsäurekonstrukte Promotoren aufweisen, die sich in ihren strangabwärts gelegenen Sequenzsegmenten unterscheiden, ferner
ein Effektor-exprimierendes Nukleinsäurekonstrukt (Effektor-exprimierende Nukleinsäurekonstrukte) umfassend, das (die) für einen Effektor codiert (codieren), wobei der Effektor an die Bindungsstellen für den Effektor der Promotoren der zwei oder mehr gewünschten Nukleinsäurekonstrukte binden kann und die Transkription ausgehend von den Promotoren aktivieren kann.

4. Eukaryotische Zelle oder Organismus nach Anspruch 3, wobei der Effektor eine Repeat-Domäne umfasst, die Repeat-Einheiten umfasst, die von einem Transcription activator-like (TAL)-Effektor stammen.

5. Eukaryotische Zelle oder Organismus nach Anspruch 3, wobei der Effektor eine RNA umfasst, die komplementär zu einer Bindungsstelle für den Effektor des Promotors ist, und die eukaryotische Zelle oder der Organismus ein Nukleinsäurekonstrukt umfasst, dass für die RNA codiert.

6. Eukaryotische Zelle oder Organismus nach Anspruch 5, wobei der Effektor eine guide RNA (gRNA) umfasst, die zu einer Bindungsstelle für den Effektor komplementär ist, wobei die Effektor-RNA bevorzugt eine single guide-RNA (sgRNA) ist, die eine crRNA und eine tracrRNA umfasst.

7. Eukaryotische Zelle oder Organismus nach einem der Ansprüche 3 bis 6, wobei eines oder mehrere der gewünschten Nukleinsäurekonstrukte eine zweite oder eine weitere Bindungsstelle für den Effektor strangaufwärts der TATA-Box umfasst.

8. Eukaryotische Zelle oder Organismus nach einem der Ansprüche 3 bis 7, wobei eines oder mehrere der gewünschten Nukleinsäurekonstrukte ein gewünschtes Nukleinsäurekonstrukt enthält, das für einen Effektor codiert, der an eine zweite oder eine weitere Bindungsstelle für einen Effektor binden kann, um die Transkription von gewünschten Nukleinsäuresequenzen in (a) einem Nukleinsäurekonstrukt (in Nukleinsäurekonstrukten), das (die) die zweite oder die weitere Bindungsstelle für den Effektor enthält (enthalten), zu Aktivieren oder zu Unterdrücken.

9. Verwendung eines Systems oder Kits zur Expression zweier oder mehrerer gewünschter Nukleinsäuresequenzen in einem eukaryotischen Organismus, wobei Menschen ausgeschlossen sind, oder in Zellen oder Gewebe eines eukaryotischen Organismus, und für das Metabolic Engineering eines Biosynthesewegs, der die gleichzeitige und abstimmbare Expression einer Vielzahl von Genen erfordert, folgendes umfassend:
(A) eine Bibliothek zahlreicher Promotoren, wobei jeder der zahlreichen Promotoren in 5'- nach 3'-Richtung folgendes umfasst:
(i) eine Bindungsstelle für einen Effektor, die wenigstens 10 zusammenhängende Basen umfasst, wobei die Basensequenzen der Bindungsstellen für den Effektor der zahlreichen Promotoren eine Sequenzidentität von wenigstens 75% aufweisen,
(ii) eine TATA-Box und
(iii) ein strangabwärts gelegenes Sequenzsegment, das wenigstens 20 zusammenhängende Basen umfasst, wobei sich die zahlreichen Promotoren in ihren strangabwärts gelegenen Sequenzsegmenten unterscheiden, und
(B) einen Effektor, der an die Bindungsstellen für den Effektor der zahlreichen Promotoren binden kann, oder ein Nukleinsäuremolekül, das für den Effektor codiert.

10. Verwendung nach Anspruch 9, wobei die Bindungsstelle für den Effektor der zahlreichen Promotoren ein Sequenzsegment von 10 bis 30 zusammenhängenden Basen ist, bevorzugt von 13 bis 27 zusammenhängenden Basen, stärker bevorzugt von 16 bis 24 zusammenhängenden Basen; und/oder
wobei die Basensequenzen der Bindungsstellen für den Effektor der zahlreichen Promotoren eine Sequenzidentität von wenigstens 85%, bevorzugt von wenigstens 90%, stärker bevorzugt von wenigstens 95% aufweisen.

11. Verwendung eines Systems oder Kits zur Expression zweier oder mehrerer gewünschter Nukleinsäuresequenzen in einem eukaryotischen Organismus, wobei Menschen ausgeschlossen sind, oder in Zellen oder Gewebe eines eukaryotischen Organismus, wobei die Expression dahingehend koordiniert wird, dass das Expressionsniveau der zwei oder mehr gewünschten Nukleinsäuresequenzen durch eine Auswahl verschiedener Promotoren aus einer Bibliothek von Promotoren vorherbestimmt wird, um das gewünschte Expressionsniveau für jede der zwei oder mehr gewünschten Nukleinsäuresequenzen zu erreichen, umfassend:
(A) eine Bibliothek zahlreicher Promotoren, wobei jeder der zahlreichen Promotoren in 5'- nach 3'-Richtung folgendes umfasst:
(i) eine Bindungsstelle für einen Effektor, die wenigstens 10 zusammenhängende Basen umfasst,
(ii) eine TATA-Box und
(iii) ein strangabwärts gelegenes Sequenzsegment, das wenigstens 20 zusammenhängende Basen umfasst, wobei sich die zahlreichen Promotoren in ihren strangabwärts gelegenen Sequenzsegmenten unterscheiden, und
wobei die Basensequenzen der Bindungsstellen für den Effektor der zahlreichen Promotoren über eine Länge von folgendem gleich sind:
10 zusammenhängenden Basen, außer, dass eine oder zwei Basen ausgetauscht werden können, oder eine Base deletiert oder inseriert werden kann und eine Base ausgetauscht werden kann, oder
15 zusammenhängenden Basen, außer, dass bis zu drei Basen ausgetauscht werden können, oder eine Base deletiert oder inseriert werden kann und eine oder zwei Basen ausgetauscht werden können, oder
20 zusammenhängenden Basen, außer, dass bis zu vier Basen ausgetauscht werden können, oder eine Base deletiert oder inseriert werden kann und bis zu drei Basen ausgetauscht werden können, und
(B) einen Effektor, der an die Bindungsstellen für den Effektor der zahlreichen Promotoren binden kann, oder ein Nukleinsäuremolekül, das für den Effektor codiert.

12. Verwendung nach einem der Ansprüche 9 bis 11, wobei die Bibliothek der Promotoren ferner ein strangaufwärts gelegenes Sequenzsegment umfasst, das sich strangaufwärts der Bindungsstelle für den Effektor befindet, wobei das strangaufwärts gelegene Sequenzsegment wenigstens 10 zusammenhängende Basen umfasst, wobei sich die zahlreichen Promotoren in dem strangaufwärts gelegenen Sequenzsegment unterscheiden können.

13. Verwendung nach einem der Ansprüche 9 bis 12, wobei die Bibliothek in einem Abschnitt vorliegt oder jeder Promotor der Bibliothek in einem separaten Abschnitt vorliegt.

## Revendications

1. Procédé pour exprimer deux ou plus de deux séquences d'acide nucléique présentant un intérêt dans des cellules eucaryotes, dans des cellules ou un tissu d'un organisme eucaryote, ou dans un organisme eucaryote à l'exclusion des humains, comprenant :
(a) l'obtention de cellules eucaryotes, de tissu d'un organisme eucaryote, ou d'un organisme eucaryote à l'exclusion des humains, comprenant deux ou plus de deux assemblages d'acide nucléique présentant un intérêt, chaque produit d'assemblage d'acide nucléique comprenant un promoteur et, en aval de celui-ci, une séquence d'acide nucléique présentant un intérêt devant être exprimée, dans laquelle chaque promoteur desdits produits d'assemblage d'acide nucléique présentant un intérêt est choisi parmi une bibliothèque de multiples promoteurs comprenant chacun, dans la direction de 5' vers 3',
(i) un site de liaison à un effecteur comprenant au moins 10 bases contiguës, dans lequel les séquences de bases de sites de liaison à un effecteur des multiples promoteurs ont une identité de séquence d'au moins 75 %,
(ii) un cadre TATA, et
(iii) un segment de séquence en aval comprenant au moins 20 bases contiguës, dans lequel les segments de séquence en aval d'au moins deux produits d'assemblage d'acide nucléique présentant un intérêt diffèrent, et
(b) la dotation aux cellules eucaryotes, au tissu d'un organisme eucaryote, ou à un organisme eucaryote de l'étape précédente, d'un effecteur capable de se lier aux sites de liaison à un effecteur des promoteurs et d'activer la transcription à partir desdits promoteurs.

2. Procédé selon la revendication 1, dans lequel l'étape (b) comprend :
la transfection des cellules eucaryotes, du tissu d'un organisme eucaryote, ou de l'organisme eucaryote, obtenus dans l'étape (a), avec un produit d'assemblage d'acide nucléique exprimant un effecteur codant ledit effecteur ; ou
l'introduction dudit effecteur dans lesdites cellules, dans des cellules dudit tissu, ou dans des cellules dudit organisme eucaryote, obtenus dans l'étape (a).

3. Cellule ou organisme eucaryote à l'exclusion des humains, comprenant deux ou plus de deux produits d'assemblage d'acide nucléique présentant un intérêt, chaque produit d'assemblage d'acide nucléique présentant un intérêt comprenant un promoteur et, en aval de celui-ci, une séquence d'acide nucléique présentant un intérêt devant être exprimée, chaque produit d'assemblage comprenant une séquence nucléique présentant un intérêt différente, dans lequel les promoteurs desdits produits d'assemblage d'acide nucléique présentant un intérêt sont choisis parmi une bibliothèque de multiples promoteurs comprenant chacun, dans la direction de 5' vers 3',
(i) un site de liaison à un effecteur comprenant au moins 10 bases contiguës, dans lequel les séquences de bases de sites de liaison à un effecteur des multiples promoteurs ont une identité de séquence d'au moins 75 %,
(ii) un cadre TATA, et
(iii) un segment de séquence en aval comprenant au moins 20 bases contiguës, sous réserve qu'au moins deux desdits deux ou plus de deux produits d'assemblage d'acide nucléique présentant un intérêt aient des promoteurs dont les segments de séquence en aval diffèrent,
comprenant en outre un ou plusieurs produits d'assemblage d'acide nucléique exprimant un effecteur codant un effecteur, dans lequel ledit effecteur peut se lier aux sites de liaison à un effecteur des promoteurs desdits deux ou plus de deux produits d'assemblage d'acide nucléique présentant un intérêt et activer la transcription à partir desdits promoteurs.

4. Cellule ou organisme eucaryote selon la revendication 3, dans lequel ledit effecteur comprend un domaine répétitif comprenant des motifs répétitifs dérivés d'un effecteur analogue à un activateur de transcription (TAL).

5. Cellule ou organisme eucaryote selon la revendication 3, dans lequel ledit effecteur comprend un ADN ayant une complémentarité vis-à-vis d'un site de liaison à un effecteur desdits promoteurs, et ledit organisme ou cellule eucaryote comprend un produit d'assemblage d'acide nucléique codant ledit ARN.

6. Cellule ou organisme eucaryote selon la revendication 5, dans lequel ledit effecteur comprend un ARN de guidage (ARNg) ayant une complémentarité vis-à-vis d'un site de liaison à un effecteur, de préférence ledit ARN d'effecteur est un ARN de guidage simple (ARNsg) comprenant un ARNcr et un ARNtracr.

7. Cellule ou organisme eucaryote selon l'une quelconque des revendications 3 à 6, dans lequel un ou plusieurs desdits produits d'assemblage d'acide nucléique présentant un intérêt comprennent un deuxième ou un autre site de liaison à un effecteur en amont du cadre TATA.

8. Cellule ou organisme eucaryote selon l'une quelconque des revendications 3 à 7, dans lequel un ou plusieurs desdits produits d'assemblage d'acide nucléique présentant un intérêt contiennent un produit d'assemblage d'acide nucléique présentant un intérêt codant un effecteur capable de se lier à un deuxième ou autre site de liaison à un effecteur pour activer ou réprimer la transcription d'une séquence d'acide nucléique présentant un intérêt dans un ou plusieurs produits d'assemblage d'acide nucléique contenant le deuxième ou autre site de liaison à un effecteur.

9. Utilisation d'un système ou d'une trousse exprimant deux ou plus de deux séquences d'acide nucléique présentant un intérêt dans un organisme eucaryote à l'exclusion des humains ou une cellule ou un tissu d'un organisme eucaryote et pour la conception métabolique d'une voie de biosynthèse requérant une expression simultanée et ajustable de multiples gènes, comprenant :
(A) une bibliothèque de multiples promoteurs, chacun desdits multiples promoteurs comprenant, dans la direction de 5' vers 3',
(i) un site de liaison à un effecteur comprenant au moins 10 bases contiguës, dans lequel les séquences de bases de sites de liaison à un effecteur des multiples promoteurs ont une identité de séquence d'au moins 75 %,
(ii) un cadre TATA, et
(iii) un segment de séquence en aval comprenant au moins 20 bases contiguës,
dans lequel les multiples promoteurs diffèrent par les segments de séquence en aval, et
(B) un effecteur capable de se lier aux sites de liaison à un effecteur desdits multiples promoteurs ; ou une molécule d'acide nucléique codant ledit effecteur.

10. Utilisation selon la revendication 9, dans laquelle le site de liaison à un effecteur des multiples promoteurs est un segment de séquence de 10 à 30 bases contiguës, de préférence de 13 à 27 bases contiguës, plus préférablement de 16 à 24 bases contiguës ; et/ou
dans laquelle les séquences de bases de sites de liaison à un effecteur des multiples promoteurs ont une identité de séquence d'au moins 85 %, de préférence d'au moins 90 %, plus préférablement d'au moins 95 %.

11. Utilisation d'un système ou d'une trousse pour exprimer deux ou plus de deux séquences d'acide nucléique présentant un intérêt dans un organisme eucaryote, dans laquelle l'expression est coordonnée en ce sens que les niveaux d'expression des deux ou plus de deux séquences d'acide nucléique présentant un intérêt sont prédéterminés par sélection de différents promoteurs à partir d'une bibliothèque de promoteurs pour que soient atteints des niveaux d'expression souhaités pour chacune des deux ou plus de deux séquences d'acide nucléique présentant un intérêt, comprenant :
(A) une bibliothèque de multiples promoteurs, chacun desdits multiples promoteurs comprenant, dans la direction de 5' vers 3',
(i) un site de liaison à un effecteur comprenant au moins 10 bases contiguës,
(ii) un cadre TATA, et
(iii) un segment de séquence en aval comprenant au moins 20 bases contiguës,
dans laquelle les multiples promoteurs diffèrent par les segments de séquence en aval ; et
dans laquelle les séquences de bases de sites se liant à un effecteur des multiples promoteurs sont les mêmes sur une longueur de
10 bases contiguës sauf qu'une ou deux bases peuvent être échangées, ou bien une base peut être supprimée ou ajoutée et une base peut être échangée, ou
15 bases contiguës sauf qu'une à trois bases peuvent être échangées, ou bien une base peut être supprimée ou ajoutée et une ou deux bases peuvent être échangées, ou
20 bases contiguës sauf qu'une à quatre bases peuvent être échangées, ou bien une base peut être supprimée ou ajoutée et une à trois bases peuvent être échangées, et
(B) un effecteur capable de se lier aux sites de liaison à un effecteur desdits multiples promoteurs ; ou une molécule d'acide nucléique codant ledit effecteur.

12. Utilisation selon l'une quelconque des revendications 9 à 11, ladite bibliothèque de promoteurs comprenant en outre un segment de séquence en amont situé en amont dudit site de liaison à un effecteur, ledit segment de séquence en amont comprenant au moins 10 bases contiguës, dans laquelle lesdits multiples promoteurs peuvent différer dans ledit segment de séquence en amont.

13. Utilisation selon l'une quelconque des revendications 9 à 12, dans laquelle ladite bibliothèque est présente dans un seul récipient, ou bien chaque promoteur de ladite bibliothèque est contenu dans un récipient séparé.
